(19) Europäisches Patentamt
European Patent Office
Office européen des brevets

(11) Publication number : 0 209 971 B1

# (12) EUROPEAN PATENT SPECIFICATION

(45) Date of publication of patent specification : 06.03.91 Bulletin 91/10

(51) Int. Cl.$^5$ : C07D 493/10, C12P 17/06, A23K 1/17, // (C07D493/10, 311:00, 307:00), (C12P17/06, C12R1:465)

(21) Application number : 86303977.2

(22) Date of filing : 27.05.86

## (54) Polyether antibiotic and process for its production.

The file contains technical information submitted after the application was filed and not included in this specification

(30) Priority : 28.05.85 US 738317
28.05.85 US 738320

(43) Date of publication of application : 28.01.87 Bulletin 87/05

(45) Publication of the grant of the patent : 06.03.91 Bulletin 91/10

(84) Designated Contracting States : AT BE CH DE FR GB IT LI NL SE

(56) References cited :
EP-A- 0 133 971
FR-A- 2 322 596

(56) References cited :
JOURNAL OF ANTIBIOTICS, vol. 33, no. 11, November 1980, pages 1224-1230, Tokyo, JP; T. MIZUTANI et al.: "Lonomycins B and C two new components of polyether antibiotics fermentation, isolation and characterization"
"POLYETHER ANTIBIOTICS", Naturally Ocurring Acid Ionophores, vol. 1, pages 1-11, chapter 1, Marcel Dekker Inc., New York, US; J.W. WESTLEY: "Notation and classification"

(73) Proprietor : ELI LILLY AND COMPANY
Lilly Corporate Center
Indianapolis Indiana 46285 (US)

(72) Inventor : Hamill, Robert L.
617 Brookview Drive
Greenwood Indiana 46142 (US)
Inventor : Yao, Raymond Che-Fong
987 Hawthorne Drive
Carmel Indiana 46032 (US)
Inventor : Nakatsukasa, Walter Mitsuo
1517 Iron Liege Road
Indianapolis, Indiana 46217 (US)

(74) Representative : Tapping, Kenneth George et al
Erl Wood Manor
Windlesham Surrey, GU20 6PH (GB)

EP 0 209 971 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to the new polyether antibiotic A80438 and to its production by the cultivation of certain novel microorganisms of the Streptomyces species.

Improved antibiotics continue to be needed in the veterinary field. Enhancing growth promotion in animals is one desired feature of such antibiotics. Growth promotion can be achieved by reducing disease and by increasing feed-utilization efficiency.

Coccidiosis is a serious problem for the poultry industry. Coccidiosis results from infection by one or more species of Eimeria or Isopora. Improved anticoccidial agents are in demand because of the continuing economic losses due to coccidiosis.

Promoting growth by increasing feed-utilization efficiency is another ecomonically desirable objective of veterinary science. Of particular interest is growth promotion in ruminants, such as cattle.

The mechanism for utilizing the major nutritive portion (carbohydrates) of ruminant feeds is well known. Microorganisms in the rumen of the animal degrade carbohydrates to produce monosaccharides and then convert these monosaccharides to pyruvate compounds. Pyruvates are metabolized by microbiological processes to form acetates, butyrates or propionates, collectively known as volatile fatty acids (VFA).

The relative efficiency of VFA utilization is connected to overall efficiency. Thus, although acetates and butyrates are used, propionates are used with greater efficiency. Also, the fermentation efficiency of propionate production is greater than that of butyrate or acetate. This is in addition to the utilization efficiency. A beneficial compound, therefore, stimulates animals to produce a higher proportion of propionates from carbohydrates, thereby increasing carbohydrate-utilization efficiency.

A80438 is a new member of the group of polyether antibiotics. Westley (John W. Westley, "Polyether Antibiotics : Naturally Occurring Acid Ionophores, Vol. 2, Chemistry," Marcel Dekker, New York, 1983) has separated existing polyethers by class and type. Using Westley's system, A80438 is a new member of the Class 1a, type (2), group of polyethers. Examples of other members of this group are : Ionomycin (also called emericid, A218, RP 31559 and DE 3936 ; see U.S. Patent 3,950,514), monensin (U.S. Patent 3,501,568), nigericin and laidlomycin.

According to the invention there is provided a new polyether antibiotic A80438 of formula 1 :

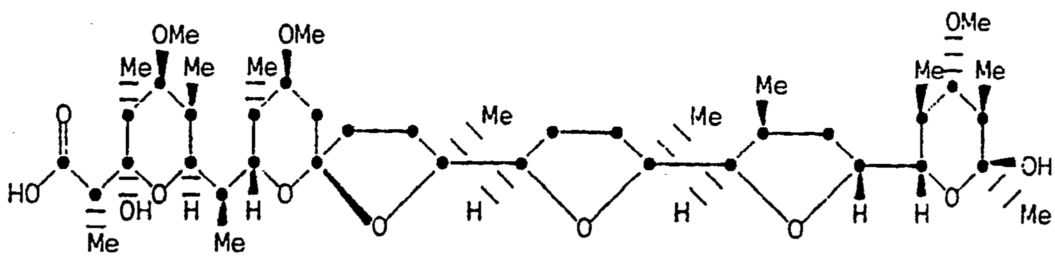

1

and its acyl, alkyl ester, alkyl ether urethane derivatives and the salts thereof.

Thus, in one aspect, this invention provides A80438 urethane derivatives which are believed to have formula 2 :

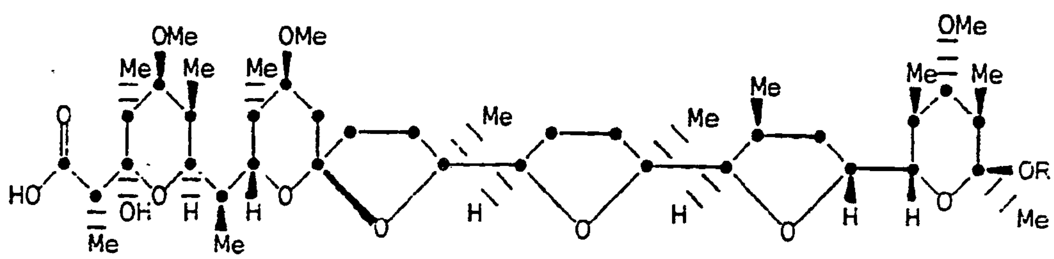

2

wherein R is $-CONHR_1$ and $R_1$ is alkyl, aryl, alkyl-aryl, arylalkyl, haloaryl, nitroaryl, haloarylalkyl,

alkoxyaryl, aryloxyaryl, arylcycloalkyl, acylaryl and cycloalkyl ; and salts of these derivatives.

A80438 is a useful antibacterial and anticoccidial agent. It improves feed-utilization efficiency in ruminants and acts as a growth promotant in ruminants and in monogastric animals. In addition, A80438 has insecticidal, inotropic and antiviral activity. Further, A80438 is useful as an ionophore.

A80438 (in its sodium salt form) has the following characteristics :

State : white crystals (from acetone-water)

mp : 160-162°C.

pKa : = 5.2 (66% aqueous dimethylformamide)

$[\alpha]^{25}D$ : + 102° (c 1, $CHCl_3$)

Molecular weight : 820 by field desorption mass spectrometry (FDMS)

Empirical formula : $C_{43}H_{74}O_{13}Na$

UV : no absorbance

IR (acid form, $CHCl_3$) : 3018, 2976, 2940, 1731, 1457, 1379, 1206, 1118, 1088, 1041, 1021, 978, and 968 $cm^{-1}$ (see Fig. 1)

IR (Na salt, $CHCl_3$) : 3018, 2979, 2938, 2881, 2832, 1710, 1591, 1454, 1387, 1306, 1243, 1215, 1170, 1117, 1089, 1076, 974, 967 and 874 $cm^{-1}$ (see Fig. 2)

Solubility : Not very soluble in water ; soluble in dimethyl sulfoxide, dimethylformamide, lower alcohols such as methanol, ketones such as acetone, esters such as ethyl acetate, halogenated hydrocarbons such as chloroform and hydrocarbons such as diethyl ether, benezene, toluene and warm hexane.

Based on its physical characteristics A80438 is believed to have the structure shown in formula 1. As is apparent from its structure, A80438 has an acid function capable of forming salts and ester derivatives. A80438 also has at least one hydroxyl group which can be esterified or which can form ether derivatives. The acyl and alkyl ester, the alkyl ether and the urethane derivatives of A80438, and the pharmaceutically-acceptable salts of A80438 and of these derivatives are also useful as antibiotics and as agents which increase feed-utilization efficiency.

The term "acyl" means a $C_1$ to $C_7$, preferably a $C_1$ to $C_4$, alkanoic acid moiety, i.e., a radical of the formula

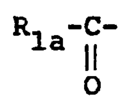

wherein $R_{1a}$ is $C_1$ to $C_6$-alkyl or hydrogen, e.g.,acetyl, propionyl, butyryl and the like.

The term "cycloalkyl" means cyclic hydrocarbon groups containing from 3 to 7 carbon atoms, such as, cyclopropyl, cyclobutyl, cyclohexyl and the like, cyclohexyl being preferred. The cycloalkyl group may be substituted by an aryl residue, as defined herein, to form an arylcycloalkyl residue, e.g., 2-(phenyl)cyclopropyl.

The term "alkoxy" means a $C_1$ to $C_7$ lower alkylgroup having an oxygen function substituted therein, such as, methoxy, ethoxy, propoxy and the like.

The term "aryl" denotes an aromatic residue derived by the removal of a hydrogen atom from an aromatic hydrocarbon, such as, for example, phenyl, pyridyl or furyl, especially phenyl. The "aryl" residue may be substituted by various groups. A substitutent on a phenyl nucleus is preferably on the 4-position. Examples are 4-alkylaryl, e.g., 4-methylphenyl (4-tolyl) ; 4-halophenyl, e.g., 4-chlorophenyl ; 4-nitrophenyl ; 4-aryloxyaryl, e.g., 4-phenoxyphenyl ; 4-alkoxyphenyl, e.g., 4-methoxyphenyl ; and 4-(alkyl-carbonyl)-phenyl, e.g., 4-(methylcarbonyl)phenyl or 4-(phenyl-carbonyl)phenyl.

The term "alkyl" means a $C_1$ to $C_7$ straight or branched chain hydrocarbon, preferably a $C_1$ to $C_4$ hydrocarbon, e.g., methyl, ethyl, propyl, isopropyl, n-butyl, etc. The alkyl group may be substituted by an aryl residue, as defined supra, to form an arylalkyl residue, e.g., phenylethyl or 2-phenylethyl or by a haloaryl residue to form a haloarylalkyl residue, e.g., 4-bromoghenethyl.

The term "an A80438 compound" is used herein to designate antibiotic A80438 (formula 1), an acyl or alkyl ester or an alkyl ether derivative of antibiotic A80438, a urethane derivative of antibiotic A80438 (formula 2) or a pharmaceutically acceptable salt of antibiotic A80438 or of its acyl or alkyl ester, alkyl ether or urethane derivatives.

Antibiotic A80438 can be produced by the submerged aerobic cultivation of an A80438-producing strain of Streptomyces bobili NRRL 15971 or Streptomyces pactum culture, NRRL 15970. The antibiotic can be recovered from the culture medium by using various isolation and purification procedures understood in the art.

Cultures of the two A80438-producing organisms have been deposited and made part of the stock culture collection of the Midwest Area Northern Regional Research Center, Agricultural Research Service, U.S.

Department of Agriculture, 1815 North University Street, Peoria, Illinois 61604, from which they are available to the public under the accession numbers NRRL 15971 (the S. bobili strain) and NRRL 15970 (the S. pactum strain).

The new Streptomyces bobili strain which is useful for the preparation of antibiotic A80438 was isolated from a soil sample from Scotland. For convenience in describing the S. bobili strain, it is called the A80438 culture.

Taxonomic studies of this organism were carried out by Frederick P. Mertz of the Lilly Research Laboratories. Based on these studies, the organism is classified as a new strain of Streptomyces bobili (Waksman and Curtis 1916) Waksman and Henrici 1948. This classification is based on an examination of published descriptions of this species (R. E. Buchanan, and N. E. Gibbons (eds), "Bergey's Manual of Determinative Bacteriology", 8th Edition, The Williams and Wilkins Co., Baltimore, 1974 ; E. G. Shirling, and D. Gottlieb, "Cooperative Description of Type Cultures of Streptomyces", Int. J. Syst. Bacteriol. 18 (4) : 279-399 (1968) ; and S. A. Waksman, "The Actinomycetes Vol. 11", The Williams and Wilkins Co., Baltimore, 1961].

## Methods Used

The methods recommended by the International Streptomyces Project (ISP) for the characterization of Streptomyces species [E. B. Shirling and D. Gottlieb, "Methods for Characterization of Streptomyces Species", Int. J. Syst. Bacteriol. 16 (3), 313-340 (1966)] have been followed along with certain supplementary tests [D. Berd, "Laboratory Identification of Clinically Important Aerobic Actinomycetes", Appl. Microbiol. 25 (4), 665-681 (1973) ; and D. J. Blazevic and G. M. Ederer, "Principles of Biochemical Tests in Diagnostic Microbiology", John Wiley and Sons, Inc., New York, 1975, p. 136).

Carbon utilization was determined on ISP No. 9 basal medium to which filter-sterilized carbon sources were added to equal a final concentration of 1.0 percent. Plates were incubated at 30°C. and read after 14 days.

Melanoid pigment production (chromogenicity) was determined with ISP No. 1 (tryptone-yeast extract broth), ISP No. 6 (peptone-yeast extract iron agar), ISP No. 7 (tyrosine agar) and modified ISP No. 7 which has tyrosine removed.

Starch hydrolysis was determined by testing for the presence of starch with iodine on ISP No. 4 (inorganic salts-starch agar) plates (see Blazevic and Ederer, supra).

Morphology was studied using an optical light microscope. A scanning electron microscope (SEM) was used to study the spore surface ornamentation.

NaCl tolerance was measured by adding NaCl to ISP No. 2 agar to equal the concentration desired.

ICSS-NBS Centroid Color Charts, standard sample No. 2106 (National Bureau of Standards, 1958, U. S. Department of Commerce, Washington, D.C.) and the Color Harmony Manual (4th ed., Color Standards Department, Container Corporation of America, Chicago, Illinois, 1958) were used to assign color names.

The isomers of diaminopimelic acid (DAP) and the carbohydrates in hydrolysates of whole cells were established by the chromatographic methods of Becker et al. [B. Becker, M. P. Lechevalier, R. E. Gordon, and H. A. Lechevalier, "Rapid Differentiation between Nocardia and Streptomyces by Paper Chromatography of Whole-cell Hydrolysates", Appl. Microbiol. 12, 421-423 (1964)] and of Lechevalier [M. P. Lechevalier, "Identification of Aerobic Actinomycetes of Clinical Importance", J. Lab. Clin. Med. 71, 934-944 (1968)].

Resistance to lysozyme was measured by methods recommended by Gordon and Barnett [R. E. Gordon and D. A. Barnett, "Resistance to Rifampin and Lysozyme of Strains of Some Species of Mycobacterium and Nocardia as a Taxonomic Tool", Int. J. Syst. Bacteriol. 27, 176-178 1977)].

Resistance to antibiotics was measured by padding antibiotic sensitivity discs onto the surface of seeded ISP No. 2 agar plates.

Phosphatase and urease were determined by methods described by Blazevic and Ederer, supra.

## Cultural Characteristics

The organism has very limited growth on both complex and chemically defined media. From a total of 15 agar culture media, only 5 support good growth, 4 support minimal growth, and the remaining 6 do not support any growth. ISP media Nos. 2, 5 and 7, Emerson's agar, and yeast-dextrose agar support good growth. Aerial mycelia are not produced. Coremia are formed abundantly. The limited growth ability and the abundant formation of coremia are distinctive features of this culture. The coremia, which macroscopically resemble poorly developed secondary growth, have a reddish color. The nearest matching color tab in the Tresner and Backus System [H. D. Tresner and E. J. Backus, "System of Color Wheels for Streptomycete Taxonomy, "Appl. Microbiol. 11 : 335-338 (1956)] is 5 cb grayish yellowish pink.

The reverse side of the colony is reddish orange to a light yellowish brown. No soluble pigments are produced. These cultural features are best demonstrated on ISP medium No. 7 (tyrosine agar). Table I presents these cultural characteristics.

## Table I

### Cultural Characteristics of A80438[a]

| Agar Medium | Characteristic[b] |
|---|---|
| ISP No. 2 | G: Good<br>R: 76.1.y Br<br>C: Poor: 5 cb g.y Pink<br>Sp: None |
| ISP No. 5 | G: Abundant<br>R: 39. gy.rO<br>C: Good: 5 gc l.rBr<br>Sp: Very light reddish brown |
| ISP No. 7 | G: Good<br>R: 39. gy.rO<br>C: Good: 5 cb g.y Pink<br>Sp: None |
| Emerson | G: Good<br>R: 76.1. yBr<br>C: Good: 5 cb g.y Pink<br>Sp: None |
| Yeast-dextrose | G: Abundant<br>R: 79. l.gy.y Br<br>C: None<br>Sp: None |

[a]On Czapek's, glycerol-glycine and potato-carrot agars, growth was poor; on ISP 3, 4, tap water, Anio-Hensens, glucose-asparagine, and tomato-paste-oatmeal agars, there was no growth.

[b]G = Growth; R = Reverse; C = Coremia; Sp = Soluble pigment.

Morphological Characteristics

A80438 does not produce sporophores. The presence of coremia represents a special morphological characteristic. The coremia are fibrous, abundant, and readily observed with both light and electron microscopes.

Physiological Characteristics

Analysis of hydrolyzed whole cells indicates that LL-DAP is present, but the meso isomer is not present. Arabinose, glucose, rhamnose, and ribose are the sugar components. The cell wall is type I. Arabinose is normally not present in Streptomyces cell walls. However, Pridham (T. G. Pridham and A. J. Lyons, Jr, "Progress in Clarification of the Taxonomic and Nomenclatural Status of Some Problem Actinomycetes", in "Developments In Industrial Microbiology, Vol. 10", American Institute of Biological Sciences, Washington, D.C., 1969) lists several species where arabinose was found. The cell-wall sugar pattern is classified "NC" (no characteristic sugar). Culture A80438 belongs to the genus Streptomyces ; however, it is not a typical taxon of this genus.

The carbon-utilization pattern for A80438 is as follows : adonitol, cellobiose, D-fructose, D-galactose, D-glucose, i-inositol, D-lactose, D-melezitose, D-melibiose, raffinose, L-rhamnose, ribose, sucrose, trehalose and D-xylose are utilized for growth. L-Arabinose, cellulose, dextran, inulin, mannitol, D-mannose, salicin and xylitol do not support growth.

Culture A80438 is resistant to cephalothin (30 µg), lincomycin (2 µg) and penicillin G (10 units) It is sensitive to bacitracin (10 units), qentamicin (10 µg), neomycin (30 µg), oleandomycin (15 units), rifampin (5 µg), streptomycin (10 µg), tetracycline (30 µg), tobramycin (10 µg), vancomycin (30 µg) and lysozyme.

Culture A80438 is Gram positive, and nonacid fast. It does not hydrolyse or peptonize skim milk, and does not liquefy gelatin. It reduces nitrate to nitrites in organic nitrate broth (ISP 8), tolerates up to 4 percent NaCl, and grows at temperatures between 10 and 42°C. It does not survive when exposed to 50°C. for 8 hours.

Melanoid pigments are not produced when A80438 is grown in ISP 1 (tryptone-yeast extract broth) or on slants of ISP 6 (peptone-yeast extract-iron agar) or ISP 7 (tyrosine agar).

A80438 produces catalase, phosphatase and urease.

Culture A80438 decomposes casein, DNA, esculin, hypoxanthine, tyrosine and xanthine. It does not decompose calcium malate, chitin, elastin, guanine, hippurate, keratin or starch.

Species Determination

Cultural, morphological, and physiological characteristics of strain A80438 were compared with the published descriptions of similar species. The following five species resemble A80438 :

Streptomyces aerocolonigenes[a]
Streptomyces bobili[a]
Streptomyces glomeroaurantiacus[b]
Streptomyces vendargenesis[c]
Streptomyces verne[c]

[a]Shirling and Gottlieb, 1968, supra.

[b]E. B. Shirling and D. Gottlieb, "Cooperative Description of Type Cultures of Streptomyces", Int. J. Syst. Bacteriol. 22 (4), 265-394 (1972).

[c]E. B. shirling and D, Gottlieb, "Cooperative Desription of Type Cultures of Streptomyces", Int. J. Syst. Bacteriol. 19 (4), 375-390 (1969).

These five cultures are reported in the literature as being devoid of aerial mycelia, not producing melanoid pigments, having a similar carbon utilization pattern, and having other cultural and physiological properties in common with A80438.

Of the five cultures, S. bobili has more characteristics in agreement with A80438. In addition, only S. bobili and S. glomeroaurantiacus are listed as validly published in the Approved Lists of Bacterial Names [V.B.D. Skerman et al., "Approved Lists of Bacterial Names", Int. J. Syst. Bacteriol. 30 (1), 225-420 (1980)].

S. bobili is similar to A80438 culturally and physiologically. Culturally, neither produces aerial mycelia or soluble pigments. Both have a rather distinctive reddish-orange pigment on the reverse side of the colony. Since neither culture produces aerial mycelia, morphological comparisons cannot be made. A80438 produces coremia. The published descriptions of S. bobili do not mention these structures. Because Waksman, supra, states that "coremia formation is of no taxonomic significance", this distinguishing feature of A80438 was not greatly emphasized.

Physiological characteristics shared by A80438 and S. bobili are : reduction of nitrate, absence of melanoid pigmentation, toleration of 4 percent NaCl, inhibition by streptomycin, and a similar carbon-utilization pattern. The similarities and differences between A80438 and S. bobili are summarized below :

| Similarities | Differences |
|---|---|
| Absence of aerial mycelium | Gelatin liquefaction |
| Absence of melanoid pigments | Growth on Czapek's agar |
| Absence of soluble pigments | Growth on glucose-asparagine agar |
| Carbon-utilization pattern | Hydrolysis of starch |
| Color of reverse side | Peptonization of milk |
| Inhibition by streptomycin | Reverse color pH indicator |
| NaCl tolerance | |
| Nitrate reduction | |

Table II gives a detailed comparison of the characteristics of A80438 and S. bobili.

Table II: Comparison of the Characteristics of A80438 and Streptomyces bobili[a]

| Characteristic | A80438 | S. bobili |
|---|---|---|
| Aerial mycelium | - | - |
| Gelatin liquefaction | - | + |
| Growth on Czapek's agar | trace | abundant |
| Growth on glucose-asparagine agar | - | + |
| Melanoid pigmentation | - | - |
| NaCl tolerance-percent | 4 | 4 |
| Nitrate reduction | + | + |
| Peptonization of skim milk | - | + |
| Reverse color a pH indicator | - | + |
| Reverse side color | reddish-orange | reddish-orange |
| Soluble pigment | - | - |
| Starch hydrolysis | - | + |
| Streptomycin inhibition | + | + |
| Utilization of: | | |
| L-arabinose | - | + |
| D-fructose | + | + |
| D-galactose | + | + |
| D-glucose | + | + |
| i-inositol | + | + |
| D-lactose | + | + |
| mannitol | - | - |
| raffinose | + | + |
| L-rhamnose | + | + |
| salicin | - | - |
| sucrose | + | + |
| D-xylose | + | + |

[a] + = Positive reaction
- = negative reaction

These comparisons demonstrate that A80438 is similar to S. bobili. The differences are not considered sufficient to erect A80438 as a new taxon. Therefore, culture A80438 is classified as a strain of Streptomyces bobili (Waksman and Curtis 1916) Waskman and Henrici 1948.

As is the case with other organisms, the characteristics of the A80438-producing culture Streptomyces bobili NRRL 15971 are subject to variation Recombinants, mutants or variants of the strain may be obtained

by methods known in the art. For example, mutants can be obtained by treatment with various known physical and chemical mutagens such as ultraviolet light, X rays, gamma rays and chemicals such as N-methyl-N'-nitro-N-nitrosoguanidine. All natural and induced variants, mutants and recombinants of this Streptomyces bobili strain which retain the characteristic of A80438 production are part of this invention.

The microorganism of this invention, which has been designated Streptomyces pactum, is useful for the preparation of antibiotic A80438. This microorganism was isolated from a soil sample from New Jersey. A culture of the Streptomyces pactum strain of this invention has been deposited and made part of the stock culture collection of the Midwest Area Northern Regional Research Center, Agricultural Research Service, U.S. Department of Agriculture, 1815 North University Street, Peoria, Illinois 61604, from which it is available to the public under the accession number NRRL 15970.

Taxonomic studies of this organism were carried out by Frederick P. Mertz of the Lilly Research Laboratories. Based on these studies, the organism is classified as a new strain of Streptomyces pactum Bhuyan, Dietz and Smith 1962. This classification is based on an examination of published descriptions of this species [R. E. Buchanan, and N. E. Gibbons (eds), "Bergey's Manual of Determinative Bacteriology", 8th Edition, The Williams and Wilkins Co., Baltimore, 1974 ; and E. B. Shirling, and D. Gottlieb, "Cooperative Description of Type Cultures of Streptomyces", Int. J. Syst. Bacteriol. 22 (4) : 265-394 (1972)].

For convenience, the new Streptomyces pactum strain is called the A80373 culture in the following discussion of its characteristics.

## Methods Used

The methods recommended by the International Streptomyces Project (ISP) for the characterization of Streptomyces species [E. B. Shirling and D. Gottlieb, "Methods for Characterization of Streptomyces Species", Int. J. Syst. Bacteriol. 16 (3), 313-340 (1966)] have been followed.

Carbon utilization was determined on ISP No. 9 basal medium to which filter-sterilized carbon sources were added to equal a final concentration of 1.0 percent. Plates were incubated at 30°C. and read after 14 days.

Melanoid pigment production (chromogenicity) was determined with ISP No. 1 (tryptone-yeast extract broth), ISP No. 6 (peptone-yeast extract iron agar) and ISP No. 7 (tyrosine agar).

Starch hydrolysis was determined by testing for the presence of starch with iodine on ISP No. 4 (inorganic salts-starch agar) plates (D. J. Blazevic and G. M. Ederer, "Principles of Biochemical Tests in Diagnostic Microbiology", John Wiley and Sons, Inc., New York, 1975, p. 136).

Morphology was studied using an optical light microscope. A scanning electron microscope (SEM) was used to study the spore surface ornamentation.

NaCl tolerance was measured by adding NaCl to ISP No. 2 agar to equal the concentration desired.

ICSS-NBS Centroid Color Charts, standard sample No. 2106 (National Bureau of Standards, U.S. Department of Commerce, Washington, D.C., 1958) and the Color Harmony Manual (4th ed., Color Standards Department, Container Corporation of America, Chicago, Illinois, 1958) were used to assign color names.

The isomer of diaminopimelic acid (DAP) and the carbohydrates in hydrolysates of whole cells were established by the chromatographic methods of Becker et al. [B. Becker, M. P. Lechevalier, R. E. Gordon, and H. A. Lechevalier, "Rapid Differentiation between Nocardia and Streptomyces by Paper Chromatography of Whole-cell Hydrolysates", Appl. Microbiol. 12, 421-423 (1964)] and of Lechevalier [M. P. Lechevalier, "Identification of Aerobic Actinomycetes of Clinical Importance", J. Lab. Clin. Med. 71, 934-944 (1968)].

Resistance to antibiotics was measured by padding antibiotic sensitivity discs onto the surface of seeded ISP No. 2 agar plates.

## Cultural Characteristics

A total of 15 agar media were used to study the cultural characteristics of this strain. Although abundant growth was observed on a number of the media, good aerial production was limited to only a few media. The culture formed aerial hyphae in the gray color series on yeast malt extract agar (ISP No. 2), glycerol asparagine agar (ISP No. 5), glucose-asparagine agar, tomato paste-oatmeal agar and tyrosine agar (ISP No. 7). The nearest matching color tab is (d) light Gray in the Tresner and Backus system [H D. Tresner and E. J. Backus, "System of Color Wheels for Streptomycete Taxonomy," Appl. Microbiol. 11 : 335-338 (1956)]. Very poor growth and aerial production were observed on oatmeal agar (ISP No. 3), Czapek's agar, Anio-Hensens agar and potato-carrot agar. Spore-mass color on these media was white. Growth was not

observed on starch salts (ISP No. 4) or tap water agar. Coremia and sterile aerial hyphae were produced on a number of agar media. A80373 is placed in the gray color series.

The reverse side of A80373 is yellow-brown to dark reddish-brown, depending on the medium. A light brown soluble pigment is present when the culture is grown in ISP Nos. 2, 5 and 7 media. The cultural characteristics on six key media are presented in Table 1.

Table 1

Cultural Characteristics of A80373 on Selected Agar Media

| Medium | | Characteristics |
|---|---|---|
| Yeast Malt. Ext. (ISP No. 2) | G: | Good |
| | R: | 75. deep y. Brown |
| | Am: | Fair: (g) medium Gray |
| | Sp: | Light brown |
| Glycerol Asparagine (ISP No. 5) | G: | Abundant |
| | R: | 47. d.gy. reddish Brown |
| | Am: | Abundant: (5fe) l.gy. r. Brown |
| | Sp: | Light brown |
| Czapek's Solution Agar | G: | Trace (excellent sporophores) |
| | R: | 263. White |
| | Am: | Trace: (a) White(scattered clumps) |
| | Sp: | None |
| Tomato Paste Oatmeal | G: | Fair |
| | R: | 94. l. olive Brown |
| | Am: | Fair: (d) light Gray |
| | Sp: | None |
| Glucose-Asparagine Agar | G: | Good (coremia) |
| | R: | 75. deep y Brown |
| | Am: | Fair: (2 ge) l. olive Brown |
| | Sp: | None |
| Tyrosine Agar (ISP No. 7) | G: | Abundant |
| | R: | 47. d.gy. reddish Brown |
| | Am: | Abundant: (2dc) y Gray |
| | Sp: | Brown |

G = Growth
R = Reverse
Am = Aerial Mycelia
Sp = Soluble Pigment

## Morphological Characteristics

A80373 produces sterile aerial hyphae on ISP No. 5 and 7 media and on glucose-asparagine agar. The culture produces coremia on Emerson, glucose-asparagine and yeast dextrose agars.

Coiled sporophores of 3-5 turns are seen on a number of media. This is best illustrated on Czapek's solution agar. Culture A80373 is placed in the Spirales (S) section of Pridham [T. G. Pridham and A. J. Lyons, Jr., "Progress in Clarification of the Taxonomic and Nomenclatural Status of Some Problem

Actinomycetes", In "Developments In Industrial Microbiology", Vol. 10, American Institute of Biological Sciences, Washington, D.C., 1969]. Spores are arranged in chains of 10 to 50.

When examined by scanning electron microscopy, the spore-surface ornamentation is either spiny (sp) or hairy (ha) with very short non-characteristics hairs.

Physiological Characteristics

Analysis of hydrolyzed whole cells indicates that LL-DAP is present, but the meso isomer is not present. Thus, A80373 has a type I cell wall (Lechevalier, supra). Sugar components of whole cells are glucose, arabinose and rhamnose. Although arabinose is normally absent from cell walls of Streptomyces, Pridham, supra, lists species where it is present. The sugar pattern is classified as NC (not characteristic). Culture A80373 is classified as belonging to the genus Streptomyces.

The carbon-utilization pattern for A80373 is as follows : A80373 uses adonitol, fructose, glucose and ribose. A80373 does not use L-arabinose, cellobiose, cellulose, dextran, galactose, inositol, inulin, lactose, mannitol, mannose, melezitose, melibiose, raffinose, L-rhamnose, salicin, sucrose, trehalose, xylitol or xylose.

Culture A80373 is resistant to cephalothin (30 µg), lincomycin (2 µg), penicillin G (10 units) and streptomycin (10 µg). It is sensitive to bacitracin (10 units), gentamicin (10 µg), neomycin (30 µg), oleandomycin (15 µg), rifampin (5 µg), tetracycline (30 µg), tobramycin (10 µg) and vancomycin (30 µg).

Culture A80373 does not reduce nitrate to nitrite in organic nitrate broth (ISP No. 8) or hydrolyze skim milk or starch. It does produce catalase, liquefy gelatin, hydrolyze calcium malate, and survive at 50°C for 8 hours. A80373 will tolerate only 1% NaCl, and will grow at temperatures between 5 and 30°C. Melanoid pigments are not produced when A80373 is grown in tryptone yeast extract broth (ISP No. 1) or on slants of peptone yeast extract iron agar (ISP No. 6) and ISP No. 7.

A comparison of A80373 and another A80438-producing strain, Streptomyces bobili NRRL 15971, indicates that, although they share many common characteristics, and both produce the same antibiotic, they are separate species. The following list summarizes this data.

Comparison of Culture A80373 and Streptomyces bobili NRRL 15971

| Similarities | Differences |
|---|---|
| Absence of melanoid pigments | Antibiotic-resistance pattern |
| Calcium malate hydrolysis | Carbon-utilization pattern |
| Catalase production | Cultural characteristics: |
| Coremia formation | Aerial hyphae |
| Skim milk reaction, negative | Reverse color |
| Starch hydrolysis, negative | Soluble pigment |
| Sugar components in cell wall | Gelatin liquefaction |
| | Morphology |
| | NaCl tolerance |
| | Nitrate reduction |
| | Spore surface ornamentation |
| | Survival at 50°C, 8 h. |
| | Temperature range |

Species Determination

Using several "keys" to the species of Streptomyces, the cultural, morphological and physiological characteristics of A80373 were used to select a group of strains with similar characteristics. The published descriptions were then examined, and compared with A80373. The following six species were selected as having close resemblance to A80373 :

Streptomyces albulus[a]
Streptomyces chattanoogensis[b]

Streptomyces craterifer[c]
Streptomyces galteri[c]
Streptomyces karnatakensis[c]
Streptomyces pactum[a]

[a]E. B. Shirling and D. Gottlieb, "Cooperative Description of Type Cultures of Streptomyces," Int. J. Syst. Bacteriol. 22 (4) : 265-394 (1972)
[b]E. B. Shirling and D. Gottlieb, "Cooperative Description of Type Cultures of Streptomyces " Int. J. Syst. Bacteriol. 18 (2) : 69-189 (1968)
[c]E. B. Shirling and D. Gottlieb, "Cooperative Description of Type Cultures of Stregtomyces" Int. J. Syst. Bacteriol. 19 (4) : 375-512 (1969)

A close inspection of the published data shows that A80373 has the most characteristics in agreement with S. karnatakensis. In addition, A80373 keyed directly to this species in several instances.

S. karnatakensis and S. pactum are clustered closely together in two independent numerical phenetic studies [W. Kurylowicz, A. Paszkiewicz, W. Woznicka, and W. Kurzatkowski, "Numerical Taxonomy of Streptomycetes," Polish Medical Publishers, Warsaw, 1975 ; and S.T. Williams, M. Goodfellow, G. Alderson, E.M.H. Wellington, P.H.A. Sneath and M.J. Sackin, "Numerical Classification of Streptomyces and Related Genera," J. Gen. Microbiol. 129, 1743-1813 (1983)]. Williams states that they are joined together at a 96% similarity level. He drops the name S. karnatakensis and uses S. pactum as the cluster name. Of the two names, only S. pactum is in the Approved Lists (V.B.D. Skerman, et al, "Approved Lists of Bacterial Names," Int. J. Syst. Bacteriol. 30 (1) : 225-420 (1980)] ; consequently, it is the only one validly published. The name S. pactum, therefore, will be used.

Culture A80373 and S. pactum both belong to the gray color series, exhibit similar reverse side color, have some soluble pigments, grow poorly on Czapek's agar, have almost identical carbon-utilization patterns, possess spiral morphology and hairy spores. Neither produces melanoid pigments. They differ in their growth on glycerol asparagine and oatmeal agars, ability to produce coremia, hydrolysis of calcium malate and NaCl tolerance.

The similarities and differences between A80373 and S. pactum are summarized below :

| Similarities | Differences |
|---|---|
| Absence of melanoid pigments | Calcium malate hydrolysis |
| Aerial spore mass color | Coremia |
| Carbon-utilization pattern | Growth on several media |
| Cultural features on Czapek's agar | NaCl tolerance |
| Reverse side color | |
| Soluble pigments | |
| Spiral sporophore morphology | |
| Spore-surface ornamentation | |

The carbon-utilization patterns of these two cultures are shown in Table 2.

Table 2

Utilization of Carbon Sources by Strain A80373 and *S. pactum* NRRL 2939

| Carbon Source | A80373 | *S. pactum* |
|---|---|---|
| None | - | - |
| L-Arabinose | - | - |
| D-Fructose | + | + |
| D-Galactose | - | + |
| D-Glucose | + | + |
| i-Inositol | - | - |
| D-Mannitol | - | - |
| Raffinose | - | - |
| L-Rhamnose | - | - |
| Salicin | - | - |
| Sucrose | - | - |
| D-Xylose | - | - |

+ = Utilized
- = Not Utilized

A listing of characteristics of A80373 is shown in Table 3.

Table 3

Physiological Characteristics of A80373

| Characteristic | A80373[a] |
|---|---|
| Catalase production | + |
| Gelatin liquefaction | + |
| Hydrolysis calcium malate | + |
| Hydrolysis skim milk | - |
| Hydrolysis starch | - |
| Melanoid pigmentation | - |
| NaCl tolerance | 1% |
| Nitrate reduction | - |
| Resistance to cephalothin | + |
| Resistance to lincomycin | + |
| Resistance to penicillin G | + |
| Resistance to streptomycin | + |
| Survival at 50°C, 8 h | + |
| Temperature range for growth | 5-30°C |
| Utilization of: | |
| adonitol | + |
| cellobiose | - |
| cellulose | - |
| dextran | - |
| inulin | - |
| lactose | - |
| mannose | - |
| melezitose | - |
| melibiose | - |
| ribose | + |
| trehalose | - |
| xylitol | - |

[a]+ = positive reaction
- = negative reaction

These comparisons demonstrate that A80373 is similar to S. pactum. Although the spore surface of A80373 is not unmistakably hairy as is S. pactum, the differences are not considered sufficient to assign A80373 to a different species. Therefore, culture A80373 is classified as a strain of Streptomyces pactum Bhuyan, Dietz and Smith 1962.

As is the case with other organisms, the characteristics of the A80438-producing culture of this invention, Streptomyces pactum NRRL 15970, are subject to variation. Recombinants, mutants or variants of the strain may be obtained by methods in the art. For example, mutants can be obtained by treatment with various known physical and chemical mutagens such as ultraviolet light, X rays, gamma rays and chemicals such as N-methyl-N'-nitro-N-nitrosoguanidine. Natural and induced variants, mutants and recombinants of Streptomyces pactum NRRL 15970 which retain the characteristic of A80438 production are part of this invention.

The culture medium used to grow Streptomyces bobili NRRL 15971 or Streptomyces pactum NRRL 15970 can be any one of a number of media. For economy in production, optimal yield, and ease of product isolation, however, certain culture media are preferred. For example, for S. bobili a preferred carbohydrate source in large-scale fermentation is glucose, although ribose, xylose, fructose, galactose, potato dextrin and the like can also be used. Glucose is also a preferred carbohydrate source for S. pactum, although sucrose, fructose, blackstrap molasses, starch and the like can also be used.

A preferred nitrogen source for both S. bobili and S. pactum is enzyme-hydrolyzed casein. Another preferred nitrogen source for S. pactum is meat peptone. Other nitrogen sources such as fish meal, liver meal, and the like should also be useful nitrogen sources.

Among the nutrient inorganic salts which may advantageously be incorporated in the culture media are the customary soluble salts capable of yielding zinc, sodium, magnesium, calcium, ammonium, chloride, carbonate, sulfate, nitrate and like ions.

Essential trace elements necessary for the growth and development of the organism should also be included in the culture medium. Such trace elements commonly occur as impurities in other substituents of the medium in amounts sufficient to meet the growth reguirements of the organism. Foaming is not usually a problem, but small amounts (i.e. 0.2 ml/L) of an antifoam agent such as polypropylene glycol may be added to large scale fermentation media if needed.

For production of substantial quantities of antibiotic A80438, submerged aerobic fermentation in tanks is preferred. Small quantities of A80438 may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger tank. The vegetative inoculum medium can be the same as that used for larger fermentations, but other media are also suitable.

A80438 is produced by Streptomyces bobili or Streptomyces pactum when grown at temperatures between about 25° and about 37°C. A good temperature for A80438 production appears to be about 30°C.

As is customary in submerged aerobic culture processes, sterile air is blown into the vessel from the bottom while the medium is stirred with conventional turbine impellors. Under the conditions used thus far, the maximum oxygen uptake of the fermentation does not exceed about 0.2 mM/L/minute. In a fully baffled 165-liter fermentor containing approximately 115 liters of broth, an aeration rate of 0.125 v/v/m with an agitation rate of 150-200 rpm is sufficient to maintain the level of dissolved oxygen at or above 30% of air saturation.

Production of antibiotic A80438 can be followed during the fermentation by testing samples of the broth for antibiotic activity against organisms known to be sensitive to the antibiotic. One assay organism useful in testing A80438 is Bacillus subtilis ATCC 6633. The bioassay is conveniently performed by the agar-well plate test.

Following its production under submerged aerobic fermentation conditions, A80438 can be recovered from the fermentation medium by methods used in the fermentation art. The antibiotic activity produced during fermentation of the A80438-producing organism occurs both in the filtered broth and in the mycelial mass. Maximum recovery of A80438 is accomplished, therefore, by initially filtering the medium to separate the broth from the mycelial mass. The filtered broth and the mycelial mass can then be purified separately to give their respective portion of A80438. A variety of techniques may be used in this purification.

A preferred technique for purification of the filtered broth involves adjusting it to a pH of about 9 and extracting with a suitable solvent such as, for example, ethyl acetate. The extracting solvent can then be evaporated under vacuum to give the broth portion of A80438.

A preferred method of purifying the mycelial mass is to extract the separated mycelial filter cake with a suitable solvent such as, for example, methanol. The extracting solvent is then evaporated under vacuum to give a concentrated aqueous solution. This aqueous solution is then adjusted to a pH of about 9 and is extracted with a suitable solvent such as, for example, ethyl acetate. The extracting solvent is then concentrated under vacuum to give the mycelial portion of A80438.

The broth and mycelial portions of the A80438 complex are further purified by similar procedures. A

preferred procedure involves silica gel chromatography.

Separation of antibiotic A80438 can be followed by thin-layer chromatography (TLC) or high performance liquid chromatography (HPLC). One convenient silica gel TLC solvent system is toluene : ethanol (4: 1). The antibiotic can be detected by bioautography using, for example, Bacillus subtilis or by other methods such as, for example, vanillin-sulfuric acid spray reagent.

Alternatively, the culture solids, including medium constituents and mycelium can be used without extraction or separation, but preferably after removal of water, as a source of A80438. For example, after production of A80438, the whole fermentation broth can be dried by lyophilization, by drum-drying, or by azeotropic distillation and drying. The dried broth is then mixed directly into feed premix.

The salts of A80438 and of its derivatives are useful for separating and purifying the antibiotics. The pharmaceutically-acceptable salts are particularly useful. Examples of salts are the alkali-metal, alkaline-earth-metal and amine salts of A80438 and of its derivatives.

Representative and suitable alkali-metal and alkaline-earth metal salts of A80438 include the sodium, potassium, lithium, cesium, rubidium, barium, calcium and magnesium salts. Suitable amine salts of A80438 include the ammonium and the primary, secondary, and tertiary $C_1$-$C_4$-alkylammonium and hydroxy-$C_2$-$C_4$-alkyl-ammonium salts. Illustrative amine salts include those formed by reaction of A80438 with ammonium hydroxide, methylamine, sec-butylamine, isopropylamine, diethylamine, di-isopropylamine, ethanolamine, triethylamine, 3-amino-1-propanol and the like.

It is well known in the veterinary pharmaceutical art that the form of an antibiotic is not ordinarily of great significance when treating an animal with the antibiotic. In most cases, conditions within the animal change the drug to a form other than that in which it was administered. The salt form in which it may be administered is, therefore, not of great significance. The salt form may, however, be chosen for reasons of economy, convenience, and toxicity.

The alkali-metal and alkaline-earth-metal cationic salts of A80438 are prepared according to procedures commonly used for the preparation of cationic salts. For example, the free acid form of A80438 is dissolved in a suitable solvent such as acetone ; about $\frac{1}{2}$ volume of water is added and this solution is adjusted to a pH of about 9 to 10 with the base of the desired cationic salt (e.g. NaOH, KOH). The salt thus formed can be isolated by routine methods, such as filtration or evaporation of the solvent.

A preferred method of forming salts is to dissolve A80438 (acid form) in a water-immiscible solvent such as ethyl acetate, add an equal volume of water ; adjust the mixture to pH 10 with the corresponding cationic base (e.g. NaOH, KOH, etc.). The separated organic phase is washed with water and concentrated to dryness. The residue is lyophilized from dioxane. The salt can be crystallized from an appropriate solvent, such as pentane.

Its formed with organic amines can be prepared in a similar manner. For example, the gaseous or liquid amine can be added to a solution of A80438 in a suitable solvent such as acetone ; the solvent and excess amine can be removed by evaporation.

A80438 acyl-ester derivatives are prepared by treating A80438 with a corresponding acid anhydride or acid halide, for example, chloride. Esterification occurs at one of the A80438 hydroxyl groups. Such esters are typically prepared by reacting A80438 with, for example, the corresponding acid anhydride at room temperature.

A80438 alkyl ester derivatives are prepared by esterification of the carboxyl group, using standard procedures. The A80438 alkyl ester derivatives are typically less active in vitro. When administered to an animal, however, such esters can act as pro drugs which are converted to A80438 in vivo.

The alkyl ether derivatives of A80438 are those compounds wherein one or more of the hydroxyl groups has been replaced by a YR group wherein :

Y represents O or S ; and

R represents $C_1$-$C_6$–alkyl,

$C_1$-$C_4$–alkoxy–$C_2$-$C_5$–alkyl,

$C_1$-$C_4$–alkoxycarbonyl–$C_2$-$C_5$–alkyl,

amino–$C_2$-$C_5$–alkyl,

mercapto–$C_2$-$C_5$–alkyl,

hydroxyalkyl,

haloalkyl, or

$(R')_m$–Phenyl$(CH_2)_n$–,

wherein R′ represents $C_1$-$C_4$–alkyl, $C_1$-$C_4$–alkoxy, or hydroxy

m represents 0-2 ; and

n represents 0-3.

The terms alkyl and alkoxy have the meaning discussed supra, but are limited to the number of carbon atoms specified.

The term "hydroxyalkyl" refers either to a monohydroxy-$C_2$-$C_5$-alkyl moiety or, when Y is O, to the 2,3-dihydroxyprop-1-yl moiety.

The term "haloalkyl" refers to a $C_2$-$C_5$-alkyl moiety having from one to three halogen substituents, selected from the group consisting of bromine, chlorine, and fluorine. When the alkyl moiety is dihalo- or trihalo-substituted, the halo-substituents must be the same halogen moiety.

Preferred A80438 ether derivatives are those compounds wherein Y represents O and R represents $C_1$-$C_6$-alkyl. The ether derivatives are prepared by reacting A80438, or a salt thereof, with a corresponding primary alcohol or thiol.

With some of the starting alcohols or thiols it may be necessary to add an acid catalyst to the reaction. Suitable catalysts include hydrochloric acid, sulfuric acid, perchloric acid, methanesulfonic acid, benzenesulfonic acid, toluenesulfonic acid, selenium dioxide, and boron trifluoride.

A solvent such as, for example, water, acetone, benzene, ether, tetrahydrofuran, or dioxane may be added to facilitate the reaction. Reactions generally occur at room temperature, although higher temperatures may be used.

Although ordinary reaction work-up procedures are sometimes sufficient, additional purification may be required to obtain the compounds of this invention. Such purification may be accomplished by well-known methods, such as, for example, column chromatography, thin-layer chromatography, fractional crystallization and the like.

The A80438 urethane derivatives of formula 2 can be prepared by treating A80438 or an A80438 salt with an isocyanate of formula 3

$$R-NCO$$

3

wherein R is as defined supra.

Preferably, a salt of A80438, in particular the sodium salt, is used. The isocyanate of formula 3 should be added in slight excess, e.g., about 10% excess, in order to form the mono derivative in optimum quantity. The reaction is preferably carried out in an inert solvent such as a chlorinated hydrocarbon, e.g., carbon tetrachloride, methylene chloride or chloroform, ether, ethyl acetate or in an aromatic hydrocarbon solvent such as benzene or toluene. The reaction temperature is not critical but can be between above 0°C. and the boiling point of the reaction mixture, but is preferably about room temperature.

The A80438 compounds inhibit the growth of bacteria and fungi which are pathogenic to animal and plant life. For example, Table III shows the inhibitory concentration (MIC) at which A80438 inhibits certain organisms. The MIC's in Table III were determined by conventional agar-dilution assays.

Table III: Antibacterial Activity of A80438 (Na salt)

| Test Organism | MIC (mcg/mL) |
|---|---|
| *Staphylococcus aureus* X1.1 | 0.5 |
| " " V41 | 0.5 |
| " " X400 | 0.5 |
| " " S13E | 0.5 |
| *Staphylococcus epidermidis* EPI1 | 0.5 |
| " " 222 | 0.25 |
| *Streptococcus pyogenes* C203 | 0.25 |
| " *pneumoniae* Park1 | 0.125 |
| " " X66 | 0.5 |
| " " 2041 | 0.5 |
| *Pseudomonas aeruginosa* X528 | 64.0 |
| *Shigella sonnei* | 16.0 |
| Other Gram-negative bacteria tested | >128 |

The A80438 compounds also are active against anaerobic bactetia. Table IV shows the MIC's at which A80438 inhibits various anaerobic bacteria, as determined by standard agar-dilution assay. End points were read after 24-hour incubation.

Table IV: Susceptibility of Anaerobic Bacterial Isolates to A80438 (Na salt)

| Anaerobic Bacteria | MIC (mcg/mL) |
|---|---|
| Clostridium difficile 2994 | 1 |
| Clostridium perfringens 81 | 1 |
| Clostridium septicum 1128 | 1 |
| Eubacterium aerofaciens 1235 | 1 |
| Peptococcus asaccharolyticus 1302 | <0.5 |
| Peptococcus prevoti 1281 | 8 |
| Peptostreptococcus anaerobius 1428 | 8 |
| Peptostreptococcus intermedius 1624 | 1 |
| Propionibacterium acnes 79 | 8 |
| Bacteroides fragilis 111 | 16 |
| Bacteroides fragilis 1877 | 16 |
| Bacteroides fragilis 1936B | 16 |
| Bacteroides thetaiotaomicron 1438 | 8 |
| Bacteroides melaninogenicus 1856/28 | 16 |
| Bacteroides melaninogenicus 2736 | 8 |
| Bacteroides vulgatis 1211 | 4 |
| Bacteroides corrodens 1874 | 16 |
| Fusobacterium symbiosum 1470 | 2 |
| Fusobacterium necrophorum 6054A | 8 |

The acute toxicity of A80438 in mice, when administered by intraperitaneal injection and expressed as $LD_{50}$, was 8.84 mg/kg.

Anticoccidial activity is an important property of the A80438 compounds. Table V summarizes the results of two in vitro tissue-culture tests in which A-80438 inhibited Eimeria tenella.

Table V: Activity of A80438 (Na salt) vs. Eimeria tenella In Vitro

| | Concentration (mcg/mL) | | | | |
|---|---|---|---|---|---|
| | 5 | 1 | 0.2 | 0.04 | 0.008 |
| Test 1 | C[a] | C | C-A | A | A |
| Test 2 | C | C | C-A | A | A |

[a]C = cytotoxic
A = active

Table VI summarizes the results of a study of the effect of A80438 in controlling an Eimeria tenella infection in chickens. In this study, groups of five 7-day-old chicks were fed a mash diet containing the antibiotic. After having been on this ration for 48 hours, each bird was inoculated with sporulated oocysts of E. tenella. Two other groups of five 7-day-old chicks were fed a mash diet which did not contain A80438. One of these groups was also inoculated with E. tenella after 48 hours (the infected controls). The other group was not inoculated with E. tenella (the normal controls). Results were evaluated seven days after inoculation. The birds were weighed, sacrificed, and examined for evidence of coccidial lesions. Coccidial involvement was measured on a scale of 0 (no evidence of coccidiosis) to 4 (maximum involvement for the Eimeria species). The percent reduction in lesion score was calculated by subtracting the average lesion score of the treated group from the average lesion score of the infected control group, dividing this difference by the average lesion score of the infected control group and multiplying by 100. The percent weight gain was calculated using the weight gain of normal controls as 100%.

Table VI: Activity of A80438 (Na Salt) vs. Eimeria tenella in vivo

| ppm in Diet | Percent Mortality | Percent Weight Gain | Percent Reduction in Lesion Scores | |
|---|---|---|---|---|
| | | | Intestinal | Cecal |
| 35 | 0.0 | 93 | 64 | 37 |
| 70 | 0.0 | 92 | 95 | 75 |
| Infected Controls | 0.0 | 79 | 0 | 0 |
| Normal Controls | 0.0 | 100 | -- | -- |

For treating coccidiosis in poultry, a non-toxic anticoccidial amount of an A80438 compound is administered to infected or susceptible birds, preferably orally on a daily basis. The A80438 compound can be supplied in many ways, but it is most conveniently supplied with a pharmaceutically acceptable carrier, preferably the feed ingested by the birds. Although a variety of factors must be considered in determining an appropriate concentration of A80438 compound, the rates of administration are generally in the range of about 2 to about 100 ppm in the feed and are preferably in the range of about 25 to about 75 ppm of feed ration.

In another aspect, this invention relates to compositions for treating coccidiosis in poultry containing an A80438 compound. One group of these are compositions comprising an effective amount of an A80438 compound for treating coccidiosis together with a suitable vehicle. Another group of these are compositions comprising an effective amount of :

1) an A80438 compound ;

in combination with

2) a compound selected from the group consisting of

a) nicarbazin,

b) 4,4'-dinitrocarbanilide,

c) a napthalenamine compound of formula 4 :

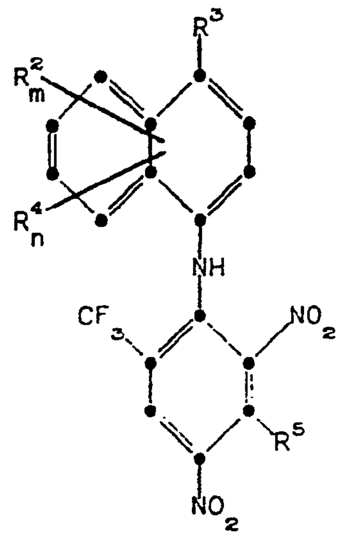

4

wherein :

$R^2$ is $C_1$-$C_4$ alkyl ;

$R^3$ is halogen, $C_1$-$C_4$ fluoroalkyl, $C_1$-$C_4$ fluoroalkoxy or $C_1$-$C_4$ fluoroalkylthio ;

$R^4$ is halogen ;

$R^5$ is hydrogen or halogen ;

m is 0, 1 or 2 ; and

n is 0 or 1 ;

with the proviso that, when an $R^4$ substituent exists, it is at other than the 2-position ;

d) a benzenamine selected from 2,4-dinitro-N-[4-(trifluoromethoxy)phenyl]-6-(trifluoromethyl)benzenamine ; 2,4-dinitro-N-[4-(1,1,2,2-tetrafluoroethoxy)-phenyl]-6-(trifluoromethyl)benzenamine or 2,4-dinitro-N-[4-(pentafluoroethoxy)phenyl]-6-(trifluoromethyl)-benzenamine ;

e) metichlorpindol ; or

f) a pharmaceutically acceptable salt of an (a)-(e) compound.

In formula 4, $C_1$-$C_4$ alkyl includes methyl, ethyl, n-propyl, isopropyl, n-butyl, sec-butyl, isobutyl, tert-butyl, and the like.

The term "halogen" represents fluorine, chlorine, bromine and iodine.

$C_1$-$C_4$ Fluoroalkyl is a $C_1$-$C_4$ alkyl group bearing one or more fluorine atoms. Such fluoroalkyl groups include trifluoromethyl, 1,1,2,2-tetrafluoroethyl, pentafluoroethyl, 1,2,3,3-tetrafluoropropyl, nonafluorobutyl, and the like.

$C_1$-$C_4$ Fluoroalkoxy is a $C_1$-$C_4$ alkoxy group bearing one or more fluorine atoms. Such fluoroalkoxy groups include difluoromethoxy, trifluoromethoxy, 1-fluoroethoxy, 1,1,2,2-tetrafluoroethoxy, pentaf-

luoroethoxy, 1,2,2,3,3-pentafluoropropoxy, heptafluoropropoxy, 4,4,4-trifluorobutoxy, and the like.

$C_1$-$C_4$ Fluoroalkylthio is a $C_1$-$C_4$ alkylthio group bearing one or more fluorine atoms. Such fluoroalkylthio groups include trifluoromethylthio, 1,1,2,2-tetrafluoroethylthio, pentafluoroethylthio, 4,4,4-trifluorobutylthio, and the like.

Preferred formula 4 compounds are those wherein m and n are 0 and $R^5$ is hydrogen.

Typical formula 4 compounds are :

4-Fluoro-N-[2,4-dinitro-6-(trifluoromethyl)-phenyl]-1-naphthalenamine
4-Iodo-N-[2,4-dinitro-6-(trifluoromethyl)-phenyl]-1-naphthalenamine
4-Trifluoromethyl-N-[2,4-dinitro-6-(trifluoromethyl)phenyl]-1-naphthalenamine
4-Pentafluoroethyl-N-[2,4-dinitro-6-(trifluoromethyl)phenyl-1-naphthalenamine
6,7-Dimethyl-4-(1,1,2,2-tetrafluoroethoxy)-N-[2,4-dinitro-6-(trifluoromethyl)phenyl]-1-naphthalenami ne
2-Isopropyl-4-chloro-N-[3-chloro-2,4-dinitro-6-(trifluoromethyl)phenyl]-1-naphthalenamine
8-n-Butyl-4-(4,4,4-trifluorobutoxy)-N-[3-bromo-2,4-dinitro-6-(trifluoromethyl)phenyl]-1-naphthalenami ne
3-Methyl-6-propyl-4-heptafluoropropyl-N-[2,4-dinitro-6-(trifluoromethyl)phenyl]-1-naphthalenamine
3,4-Dichloro-N-[2,4-dinitro-6-(trifluoromethyl)phenyl]-1-naphthalenamine
4-(1,1-Difluoroethoxy)-N-[2,4-dinitro-6-(trifluoromethyl)phenyl]-1-naphthalenamine
4-(1,1,2,2-Tetrafluoroethoxy)-N-[2,4-dinitro-6-(trifluoromethyl)phenyl]-1-naphthalenamine and
4-(1,1,2,2-Tetrafluoroethylthio)-N-[2,4-dinitro-6-(trifluoromethyl)phenyl]-1-naphthalenamine.

Anticoccidial combinations comprising nicarbazin or 4,4'-dinitrocarbanilide and polyether antibiotics are disclosed by Maurice E. Callender and Thomas K. Jeffers in U.S. Patent 4,218,438, issued August 19, 1980. Anticoccidial combinations of the napthalenamines of formula 4 with polyether antibiotics are disclosed by Albert J. Clinton and George O. P. O'Doherty in copending application Serial No. 631,665, filed July 17, 1984. Anticoccidial combinations comprising the specified benzenamines and a polyether antibiotic are disclosed by Clinton and O'Doherty in U.S. Patent 4,366,168. A coccidiocidal combination of monensin (a polyether antibiotic) and metichlorpindol is disclosed by Larry R. McDougald in U.S. Patent 4,061,755, issued December 6, 1977.

Nicarbazin and 4,4'-dinitrocarbanilide are taught in U.S. Patent 2,731,382. Nicarbazin is a complex of 4,4'-dinitrocarbanilide and 2-hydroxy-4,6-dimethylpyrimidine, but the 4,4'-dinitrocarbanilide alone exhibits anticoccidial activity. See Science 122, 244 (1955).

The components of the combinations of an A80438 compound with compounds 2(a)-(e) are used in amounts which, in combination, are synergistic as to at least one coccidiosis-causing organism. In general, the maximum amounts to be used in the combinations are the same as the maximum amounts for anticoccidial treatment by the individual components. The lower limits are generally less than that required for therapy by the individual components. Accordingly, the present invention is generally practiced with compositions containing

1) from about 2 to about 100 ppm of an A80438 compound and
2)
   a) from about 5 to 125 ppm of nicarbazin,
   b) from about 25 to about 150 ppm of 4,4'-dinitrocarbanilide,
   c) from about 1 to about 1000 ppm of the specified napthalenamine,
   d) from about 5 to about 125 ppm of a specified benzenamine, or
   e) from about 20 to about 70 ppm of metichlorpindol.

The A-80438 compounds should be particularly effective when administered with nicarbazin. Preferred combinations contain from about 2 to about 20 ppm of an A80438 compound with from about 5 to about 50 ppm of nicarbazin.

Another important property of the A80438 compounds is the ability to improve feed-utilization efficiency in animals. For example, the A80438 compounds improve feed-utilization efficiency in ruminants which have a developed rumen function.

The efficiency of feed use can be monitored by observing the production and concentration of propionate compounds in the rumen using the method described by Arthur P. Raun in U.S. Patent 3,794,732 (see especially Example 5). Table VII shows the ratio of volatile-fatty-acid (VFA) concentrations in A80438-treated flasks to concentrations in control flasks in this test.

Table VII:

Effect of A80438 on Ruminant Feed-Utilization Efficiency

| | Ratio of Treated to Control Values | | | |
|---|---|---|---|---|
| Dosage mcg/mL | Molar % Propionate | Molar % Acetate | Molar % Butyrate | Total VFA mM/L |
| 5 | 1.519* | 0.892 | 0.582 | 0.934 |
| 1 | 1.367* | 0.921 | 0.710 | 0.919 |
| 0.2 | 1.262* | 0.863 | 0.933 | 1.013 |

*LSD; two-tailed t-test; significant at P<0.01; $C_3$ >99 percent upper confidence limit

The A80438 compounds are typically effective in increasing propionate and, thereby, the efficiency of feed utilization when administered to ruminants orally at rates of from about 0.02 mg/kg/day to about 1.5 mg/kg/day. Preferable rates of administration are from about 0.05 mg/kg/day to about 0.5 mg/kg/day. A preferred method of administration is to mix the compound with the animals' feed.

This invention further relates to feed compositions adapted to increase feed utilization in ruminant animals comprising feed ration and from 2.5 to 25 grams per ton of an A80438 compound.

As described supra, A80438 compounds are active against anaerobic bacteria, including Clostridium perfringens. A80438 compounds should, therefore, be beneficial in the treatment of (which includes prevention of) enteritis in chickens, swine, cattle and sheep. A80438 compounds should also be useful in the treatment of enterotoxemia in ruminants.

In another aspect the A80438 compounds are useful in the treatment of swine dysentery. A80438 inhibits the growth of Treponema hyodysenteriae, the causative agent of swine dysentery, at levels less than or equal to 0.39 mcg/ml.

A preferred method of administering an A80438 compound to swine comprises incorporating an effective amount of the compound into the feed ration or drinking water. An appropriate effective amount will depend upon whether the treatment is to prevent dysentery in swine exposed to the disease or to cure swine already infected with the disease. Usually, a lower concentration of active compound is needed to prevent infection than is required to eliminate infection in animals already afflicted. In general, amounts in the range of from about 20 to about 100 grams of A80438 compound per ton of feed are effective to prevent infection. Amounts in the range of from about 50 to about 250 grams of A80438 compound per ton of feed are recommended for treating swine suffering from dysentery. These amounts provide from about 1 to about 5 mg/kg of body weight per day (prophylactic treatment) or from about 2.5 to about 12.5 mg/kg of body weight per day (treatment of infected animal). When added to the drinking water, amounts of from about 0.04 to about 0.2 g (prophylactic) or from about 0.2 to about 1 g (therapeutic) of A80438 compound per gallon of water are recommended.

This invention further relates to feed compositions for treating swine dysentery comprising swine ration and an effective amount of an A80438 compound. As discussed supra, an effective amount is typically one in the range of from about 20 to about 250 grams of A80438 compound per ton of feed.

The A80438 compounds are active against polio virus. For example, tissue-culture tests show that A80438 is active against Polio III virus at a level of 2000 mcg/mL.

Antibiotic A80438 exhibits ion-transport properties and is, therefore, an ionophore (ion-bearer) (see B. C. Pressman in "Inorganic Biochemistry", G. L. Eichhorn, Ed. ; Elsevier, new York, 1973 ; Vol. 1, Chapter 6). At a 0.5 mcg/mL concentration A80438 showed ionophorous activity ; its activity with $na^+$, $K^+$ and $Rb^+$ was better than that with $Cs^+$ or $Li^+$. At a lower concentration (0.2 mcg/mL), A80438 appeared to select $Na^+$ over other alkali-metal cations.

A80438 can be used, therefore, when the selective removal of particular cations is desired. Examples of such uses include the removal and recovery of silver ions from solutions in photography, the removal of toxic cations from industrial waste streams before such streams are discharged to the environment, and desalinization of sea water. A80438 can be used as one component of an ion-specific electrode (see 0. Kedem, et al., U.S. Patent 3,753,887, Aug. 21, 1973).

A80438 alters the cation permeability of both natural and artificial membranes. A80438 can be used,

therefore, as a component in a membrane used for the selective transport of cations against a concentration gradient. One potential application of this property is in recovery of heavy and precious metals on a commercial basis [see E. L. Cussler, D. F. Evans, and Sister M. A. Matesick, Science 172, 377 (1971)].

In yet another aspect, A80438 is active as an inhibitor of the enzyme ATPase. ATPase, an alkali-metal-sensitive enzyme found in cell membranes, is involved in the energy necessary for active transport. "Active transport" refers to the energy requiring series of operations whereby intracellular and extracellular fluids maintain their compositions. Inhibitors of ATPase reduce the energy required for active transport. In vitro tests have shown that A80438 inhibits mitochondrial ATPase with a half-effective concentration ($IC_{50}$) of 0.5 mcg/mL.

The A80438 compounds are also potential cardiotonic agents.

The A80438 compounds can be administered to animals orally or parenterally. The most practical way to administer the A80438 compounds is by formulation into the feed supply. A variety of feeds, including the common dry feeds, liquid feeds, and pelleted feeds, may be used. Although the preferred method of administration is by mixing it with the animals' feed, it can also be administered in other ways, for example, tablets, drenches, boluses, or capsules. Each individual dosage unit should contain a quantity of A80438 compound directly related to the proper daily dose for the animal to be treated.

The methods of formulating drugs into animal feeds are well known. A preferred method is to make a concentrated drug premix which in turn is used to prepare medicated feeds. Typical premixes may contain from about 1 to about 200 grams of drug per pound of premix. Premixes may be either liquid or solid preparations.

The final formulation of feeds for animals will depend upon the amount of drug to be administered. The common methods of formulating, mixing, an pelleting feeds may be used to prepare feeds containing an A80438 compound.

The A80438 compounds may be formulated for parenteral administration by methods recognized in the veterinary pharmaceutical art. Effective injectable compositions containing the A80438 compounds may be in either suspension or solution form. In the solution form, the A80438 compound is dissolved in a physiologically acceptable carrier. Such carriers comprise a suitable solvent, preservatives such as benzyl alcohol, if needed, and buffers. Useful solvents include, for example, alcohols, glycols, or inert oils such as vegetable oils or highly refined mineral oils.

Injectable suspension compositions are prepared using a nonsolvent for the compound with adjuvants, as a carrier. The nonsolvent can be, for example, water or a glycol such as polyethylene glycol.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful for suspending the compounds. Lecithin, alkylphenol polyethylene oxide adducts, naphthalenesulfonates, alkylbenzenesulfonates, and the polyoxyethylene sorbitan esters are useful suspending agents in liquid nonsolvents.

Many substances which affect the hydrophilicity, density, and surface tension of the liquid nonsolvent can assist in making injectable suspensions in individual cases. For example, silicone antifoams, glycols, sorbitol, and sugars can be useful suspending agents.

In order to illustrate more fully the operation of this invention, the following examples are provided :

## Example 1

### Preparation of Antibiotic A80438 Using Streptomyces bobili

### A. Shake-flask Fermentation of Streptomyces bobili

The culture Streptomyces bobili NRRL 15971, either as a lyophilized pellet or as a suspension maintained in liquid nitrogen, is used to inoculate a vegetative medium having the following composition :

Vegetative or Seed Medium

| Ingredient | Amount (g/L) |
|---|---|
| Glucose | 15 |
| Potato Dextrin | 10 |
| $Na_2SO_4$ | 2 |
| Pancreatic Digest of Casein* | 5 |
| Yeast Extract | 5 |
| Tap water | q.s. 1 liter |

*Bacto Tryptone (Difco Laboratories, Detroit, Mich.)

Slants or plates are prepared by adding 2.5% agar to the seed medium. The inoculated slant is incubated at 30°C. for from about 10 to about 14 days. The mature slant culture is scraped with a sterile tool to loosen the spores and remove and macerate the mycelial mat. About one-fourth of the loosened spores and culture growth thus obtained is used to inoculate 50 mL of a first-stage seed medium.

The inoculated first-stage medium is incubated in a 250-mL Erlenmeyer flask at 30°C. for about 72 hours on a shaker orbiting in a two-inch (5.08 cm) circle at 250 rpm.

This incubated first-stage medium (1.25 mL) is used to inoculate 50 mL of a production medium having the following composition :

| Ingredient | Amount (g/L) |
|---|---|
| Glucose | 40.0 |
| Soybean flour | 7.5 |
| Enzyme-hydrolyzed casein* | 3.0 |
| $Na_2SO_4$ | 2.0 |
| $CaCO_3$ | 2.0 |
| Tap water | q.s. 1 liter |

*Amber EHC (Amber Laboratories, Juneau, Wisc.)

The inoculated production medium is incubated in a 250-mL wide-mouth Erlenmeyer flask at 30°C. for 8 to 10 days on a shaker orbiting in a two-inch circle at 250 rpm.

B. Tank Fermentation of Streptomyces bobili

In order to provide a large volume of inoculum, 10 mL of incubated first-stage medium, prepared as described in Section A, is used to inoculate 400 mL of a second-stage growth medium having the same composition as that of the first-stage medium. This second-stage vegetative medium is incubated in a two-liter wide-mouth Erlenmeyer flask for about 48 hours at 30°C. on a shaker orbiting in a two-inch circle at 250 rpm.

Incubated second-stage vegetative medium (400 mL) thus prepared is used to inoculated 100 liters of

sterile production medium, prepared as described in Section A. The inoculated production medium is allowed to ferment in a 165-liter stirred fermentation tank for 8 to 10 days at a temperature of 30°C. Low airflow (0.12-0.25 v/v/m) and low rpm (150-200) in the stirred vessel maintain a dissolved oxygen level above 30% of air saturation.

Example 2

Isolation of A80438 produced by Streptomyces bobili

Whole fermentation broth (200 L) containing 3% Hyflo Supercel was filtered through a filter press. The mycelial cake was extracted twice by circulating methanol (50-60 L) through the press.

This extract was concentrated under vacuum to remove the methanol. The resulting aqueous solution (38 L), adjusted to pH 8 with NaOH, was extracted twice with ethyl acetate(1/3 volume).

The broth filtrate, adjusted to pH 8 with NaOH, was extracted twice with ethyl acetate (1/2 volumes). The ethyl acetate extracts were combined and concentrated under vacuum to a volume of 1 L.

The broth and mycelial ethyl acetate extracts were combined and concentrated under vacuum to a volume of 1 L.

Extracts obtained by this procedure from 400 L of whole fermentation broth were combined and concentrated under vacuum to an oily residue. The residue, dissolved in toluene (400 mL), was applied to a column containing silica gel (Woelm, 2 L) packed in toluene. The column was developed sequentially with toluene (6 L) and toluene : ethyl acetate mixtures [9 : 1 (6 L), 4 : 1 (8 L) and 3 : 2 (4 L)]. Fractions having a volume of 1 L were collected during elution of the first 12 L, and 500-ml fractions were collected thereafter. Elution was followed by

1) bioassay using Bacillus subtilis and

2) silica gel TLC (Merck plates), using a toluene : ethanol (4 : 1) solvent system and vanillin–$H_2SO_4$ spray for detection. Fractions containing A80438 [from the toluene : ethyl acetate (4 : 1) eluate] were combined and concentrated under vacuum to a volume of 80 mL.

A portion of this concentrate (10 mL) was applied to a column containing 70 mL of silica gel (Woelm 100-200) packed in toluene. This column was developed sequentially with toluene : ethyl acetate (9 : 1, 4: 1, and 3 : 2), collecting 10-mL fractions. Elution was monitored by TLC. Fractions containing A80438 were combined and concentrated under vacuum to dryness. The residue was dissolved in a small quantity of dioxane ; water (1/4 volume) was added ; and the solution was freeze-dried to give 257 mg of amorphous A80438 as a powder. The powder was dissolved in acetone (3.5 mL) ; water (1.5 mL) was added ; and the mixture was allowed to stand at 5°C overnight. The crystals which formed were separated by filtration, washed with acetone : water (1 : 1) and dried under vacuum to give 233 mg of crystalline A80438.

Example 3

Preparation of Antibiotic A80438 Using Streptomyces pactum

A. Shake-flask Fermentation of Streptomyces pactum

The culture Streptomyces pactum NRRL 15970, either as a lyophilized pellet or as a suspension maintained in liquid nitrogen, is used to inoculate an agar slant having the following composition :

Agar Slant Medium

| Ingredient | Amount (g/L) |
|---|---|
| Glucose | 2.5 |
| Soluble Starch | 5.0 |
| Yeast Extract | 1.25 |
| Enzyme-hydrolyzed Casein* | 1.25 |
| $CaCO_3$ | 0.25 |
| Agar | 15.0 |
| Deionized Water | q.s. to 1 liter |

Unadjusted pH = 6.3; adjust to pH 7.5 with NaOH; pH after sterilization = 6.9

*N-Z Amine A, Humko-Sheffield Chemical, Lyndhurst NJ

The inoculated slant is incubated at 30°C. for from about 10 to about 14 days. The mature slant culture is scraped with a sterile tool to loosen the spores and remove and macerate the mycelial mat. About one-fourth of the loosened spores and culture growth thus obtained is used to inoculate 50 mL of a first-stage vegetative medium having the following composition :

Vegetative Medium

| Ingredient | Amount (g/L) |
|---|---|
| Glucose | 10 |
| Soluble Starch | 20 |
| Enzyme-hydrolyzed Casein* | 5 |
| Yeast Extract | 5 |
| $CaCO_3$ | 1 |
| Tap water | q.s. 1 liter |

Unadjusted pH = 6.6; adjust to pH 7.2 with NaOH; pH after sterilization = 6.8

*N-Z Amine A

The inoculated first-stage medium is incubated in a 250-mL Erlenmeyer flask at 30°C. for about 48 hours on a shaker orbiting in a two-inch (5.08 cm) circle at 250 rpm.

This incubated first-stage medium (1.25 mL) is used to inoculate 50 mL of a production medium having the following composition :

| Ingredient | Amount |
|---|---|
| Glucose | 20 g/L |
| Blackstrap Molasses | 20 g/L |
| Peptone* | 5 g/L |
| $CaCO_3$ | 2 g/L |
| Czapek's Mineral Stock** | 2 mL |
| Deionized water | q.s. 1 liter |

*Bacto Peptone (Difco Laboratories, Detroit MI)

**Czapek's Mineral Stock has the following composition:

| | |
|---|---|
| KCl | 10% |
| $MgSO_4 \cdot 7H_2O$ | 10% |
| $FeSO_4 \cdot 7H_2O$ | 0.2% (dissolved in 2 ml of conc. HCl) |
| Deionized water | q.s. to 1 liter |

The inoculated production medium is incubated in a 250-mL wide-mouth Erlenmeyer flask at 30°C. for 8 to 10 days on a shaker orbiting in a two-inch circle at 250 rpm.

B. Tank Fermentation of Streptomyces pactum

In order to provide a large volume of inoculum, 10 mL of incubated first-stage medium, prepared as described in Section A, is used to inoculate 400 mL of a second-stage growth medium having the same composition as that of the first-stage medium. This second-stage vegetative medium is incubated in a two-liter wide-mouth Erlenmeyer flask for about 48 hours at 30°C. on a shaker orbiting in a two-inch circle at 250 rpm.

Incubated second-stage vegetative medium (400 mL) thus prepared is used to inoculate 100 liters of sterile production medium, prepared as described in Section A. The inoculated production medium is allowed to ferment in a 165-liter stirred fermentation tank for 6 to 8 days at a temperature of 30°C. Low airflow (0.125 v/v/m) and low rpm (150-175) in the stirred vessel maintain a dissolved oxygen level above 30% of air saturation.

Example 4

Isolation of A80438 Produced by Streptomyces pactum

Whole fermentation broth (114 L), prepared as described in Example 3 and mixed with Hyflo Supercel (3%), was adjusted to pH 3.0 with HCl and filtered through a filter press. The mycelial cake was extracted twice in the press with a solution (30 L) of methanol : water (9 : 1) containing 1% $NaHCO_3$. The methanol extracts were combined (60 L), concentrated under vacuum to a volume of 14 L, and adjusted to pH 8.5 with NaOH. The concentrate was extracted twice with ethyl acetate (1/2 volumes), and the combined extracts (12 L) were concentrated under vacuum to a volume of 0.6 L.

Concentrates obtained in this manner from three 100-L fermentations were combined and evaporated to dryness under vacuum. The residue, dissolved in $CHCl_3$ (200 ml), was applied to a 6- × 40-cm column of silica gel (1 L, Woelm 100-200 µm) packed in $CHCl_3$. The column was eluted with $CHCl_3$ (6 L) and $CHCl_3$:EtOAc [49 : 1 (1 L) and 9 : 1 (4 L)]. Elution was monitored by TLC and bioassay. Fractions containing A80438 were combined and concentrated under vacuum to dryness.

The residue thus obtained, dissolved in acetone (150 mL), was applied to small amount of silica gel (Woelm 100-200 µm) and dried. The silica gel containing A80438 was then applied to a 6- × 40-cm column

containing silica gel (1 L, Woelm 100-200 μm) packed in $CHCl_3$. The column was developed sequentially with $CHCl_3$ (6 L) and $CHCl_3$ : EtOAc [9 : 1 (5 L) and 4 : 1 (2 L)]. Elution was monitored by TLC and bioassay. Fractions containing A80438 were combined and concentrated under vacuum to dryness. The residue obtained was crystallized from acetone to give 14.27 g of crystalline A80438 as the sodium salt (see Figure 2 of the attached drawings).

Example 5

Preparation A80438 Free Acid from A80438 Na

A80438 sodium salt (1 g) was dissolved in acetone (5 mL) ; water (25 mL) was added ; and the pH of this solution was adjusted to 3.0 with 1 N HCl. Water (25 mL) was added, and a gummy precipitate formed. The mixture was extracted twice with ethyl acetate (100 mL each). The combined extracts were evaporated under vacuum to dryness. The residue was dissolved in acetone (50 mL), and water (25 mL) was added. The mixture again was allowed to stand at room temperature until the gummy precipitate formed. The precipitate was separated and dissolved in warm pentane (100 mL). The pentane solution was allowed to stand at room temperature. The pentane was then decanted and evaporated under vacuum to give 849 mg of A80438 in the acid form (mp 55-56°C) as a white powder (see Figure 1 of the attached drawings).

Example 6

Preparation of A80438 Potassium Salt

A80438 (acid form, 400 mg) was dissolved in acetone (50 mL) ; water (25 mL) was added ; and the pH was adjusted to 10.0 with 1 N KOH. Water (25 mL) was added, and the mixture was stirred for 15 minutes, maintaining the pH at 10. The mixture was then extracted twice ethyl acetate (100 mL each), and the combined extracts were evaporated under vacuum under dryness. The residue was dissolved in acetone (50 mL) ; water (50 mL) was added ; and the mixture was allowed to stand at room temperature overnight. The crystals which formed were separated by filtration, washed with water and dried in a vacuum oven at 50°C to give 179 mg of A80438 potassium salt (mp 157-159°C).

**Claims**

**Claims for the contracting States : BE, CH, DE, FR, GB, IT, LI, NL, SE**

1. Antibiotic A80438 of the formula 1 :

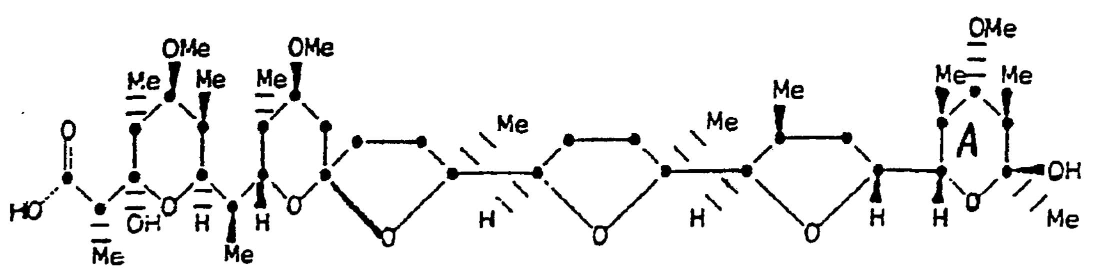

( 1 )

or

i) the $C_1$-$C_7$ acyl ester derivative of one of the hydroxyl groups

or

ii) the derivative wherein one or more of the hydroxyl groups has been replaced by a YR group wherein

Y = O or S

R = $C_1$-$C_6$ alkyl, $C_1$-$C_4$–alkoxy–$C_2$-$C_5$alkyl, $C_1$-$C_4$–alkoxycarbonyl–$C_2$-$C_5$–alkyl, amino–$C_2$-$C_5$–alkyl, mercapto–$C_2$-$C_5$–alkyl, monohydroxy–$C_2$-$C_5$–alkyl, halo–$C_2$-$C_5$–alkyl or $(R')_m$–phenyl$(CH_2)_n$–, wherein R′ represents $C_1$-$C_4$ alkyl, $C_1$-$C_4$ alkoxy or hydroxy, m = 0-2 and n = 0-3, and when Y = O, R

may also be 2,3-dihydroxyprop-1-yl or

iii) the $C_1$-$C_7$ alkyl, aryl–$C_1$-$C_7$–alkyl or haloaryl–$C_1$-$C_7$–alkylester derivative of the carboxyl group, or

iv) the urethane derivative of the A-ring hydroxyl group whereby the OH is replaced by $OR^2$, where $R^2$ = $C_1$-$C_7$ alkyl, aryl, $C_1$-$C_7$ alkylaryl, aryl–$C_1$-$C_7$–alkyl, haloaryl, nitroaryl, haloaryl–$C_1$-$C_7$–alkyl, $C_1$-$C_7$ alkoxyaryl, aryloxyaryl, aryl–$C_3$-$C_7$ cycloalkyl, $C_1$-$C_7$ acylaryl or $C_3$-$C_7$ cycloakyl or

a salt thereof, whereby aryl in any of the above definitions may be phenyl, furyl or pyridyl.

2. Antibiotic A80438 of formula 1 according to claim 1.

3. The sodium salt of antibiotic A80438 of formula 1 according to claim 1.

4. A compound of formula 1 as claimed in any one of claims 1 to 3, for use in preventing or treating microbial infections.

5. A compound of formula 1 as claimed in any one of claims 1 to 3, for use as a growth promotor.

6. A process for preparing a compound of formula 1, as claimed in any one of claims 1 to 3, which comprises cultivating Streptomyces bobili NRRL 15971, or Streptomyces pactum NRRL 15970, or an A80438-producing variant of mutant thereof, in a culture medium containing assimilable sources of carbon, nitrogen, and inorganic salts under submerged aerobic fermentation conditions until antibiotic A80438 is produced, optionally followed by separating A80438 from the culture medium.

7. Streptomyces bobili NRRL 15971.

8. Streptomyces pactum NRRL 15970.

9. A poultry feedstuff composition comprising a first component which is a compound of formula 1 as claimed in any one of claims 1 to 3 and a second component which is selected from the group consisting of

a) nicarbazin,

b) 4,4′-dinitrocarbanilide,

c) a naphthalenamine of the formula

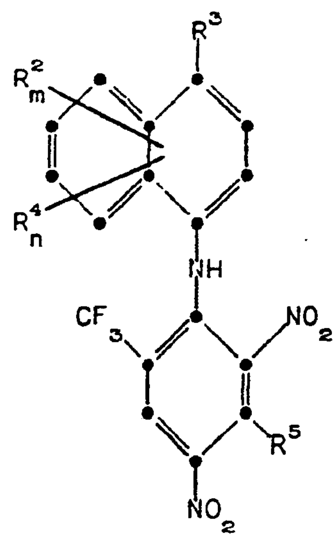

wherein :

$R^2$ is $C_1$-$C_4$ alkyl ;

$R^3$ is halogen, $C_1$-$C_4$ fluoroalkyl, $C_1$-$C_4$ fluoroalkoxy or $C_1$-$C_4$ fluoroalkylthio ;

$R^4$ is halogen ;

$R^5$ is hydrogen or halogen ;

m is 0, 1 or 2 ; and

n is 0 or 1 ;

with the proviso that, when an $R^4$ substituent exists, it is at other than the 2-position ;

d) a benzenamine selected from 2,4-dinitro-N-[4-(trifluoromethoxy)phenyl]-6-(trifluoromethyl)benzenamine, 2,4-dinitro-N-[4-(1,1,2,2-tetrafluoroethoxy)-phenyl]-6-(trifluoromethyl)benzenamine or 2,4-dinitro-N-[4-(pentafluoroethoxy)phenyl]-6-(trifluoromethyl)-benzenamine ;

e) metichlorpindol ; or

f) a pharmaceutically acceptable salt of an (a)-(e) compound ;

the components being present in the feedstuff in amounts which, in combination, are synergistic as to at least one coccidiosis-causing strain of Eimeria.

**Claims for the following Contracting State : AT**

1. A process for preparing antibiotic A80438 of the formula 1 :

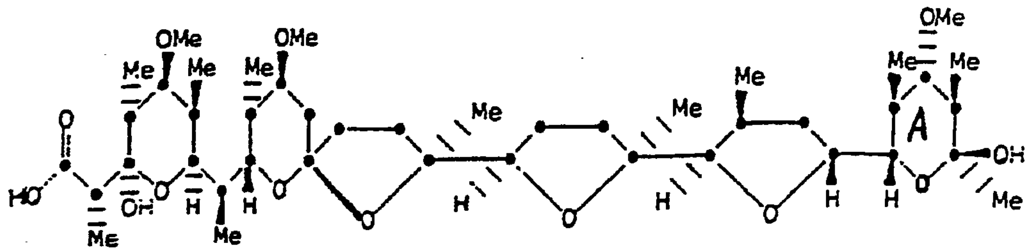

(1)

or
i) the $C_1$-$C_7$ acyl ester derivative of one of the hydroxyl groups
or

ii) the derivative wherein one or more of the hydroxyl groups has been replaced by a YR group wherein
Y = O or S
R = $C_1$-$C_8$ alkyl, $C_1$-$C_4$–alkoxy–$C_2$-$C_5$ alkyl, $C_1$-$C_4$–alkoxycarbonyl–$C_2$-$C_5$–alkyl, amino–$C_2$-$C_5$–alkyl, mercapto–$C_2$-$C_5$–alkyl, monohydroxy–$C_2$-$C_5$–alkyl, halo–$C_2$-$C_6$–alkyl or $(R')_m$–phenyl $(CH_2)_n$–, wherein R'represents $C_1$-$C_4$ alkyl, $C_1$-$C_4$ alkoxy or hydroxy, m = 0-2 and n = 0-3, and when Y = 0, R may also be 2,3-dihydroxyprop-1-yl or
iii) the $C_1$-$C_7$ alkyl, aryl-$C_1$-$C_7$–alkyl or haloaryl-$C_1$-$C_7$–alkylester derivative of the carboxyl group, or
iv)the urethane derivative of the A-ring hydroxyl group whereby the OH is replaced by $OR^2$, where $R^2$ = $C_1$-$C_7$ alkyl, aryl, $C_1$-$C_7$ alkylaryl, aryl–$C_1$-$C_7$–alkyl, haloaryl, nitroaryl, haloaryl–$C_1$-$C_7$–alkyl, $C_1$-$C_7$ alkoxyaryl, aryloxyaryl, aryl-$C_3$-$C_7$ cycloalkyl, $C_1$-$C_7$ acylaryl or $C_3$-$C_7$ cycloalkyl or
a salt thereof, whereby aryl in any of the above definitions may be phenyl, furyl or pyridyl, which comprises cultivating Streptomyces bobili NRRL 15971, or Streptomyces pactum NRRL 15970, or an A80438-producing variant or mutant thereof, in a culture medium containing assimilable sources of carbon, nitrogen, and inorganic salts under submerged aerobic fermentation conditions until antibiotic A80438 is produced, optionally followed by separating A80438 from the culture medium.

2. A process according to claim 1 for preparing A80438 as its sodium salt.

## Ansprüche

**Patentansprüche für die vertragsstaaten : BE, CH, DE, FR, GB, IT, LI, NL, SE**

1. Antibiotikum A80438 der Formel 1 :

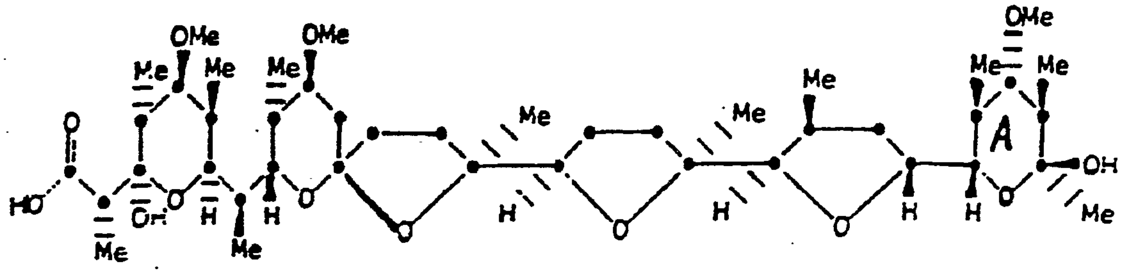

(1)

oder

i) das $C_1$-$C_7$-Acylesterderivat einer der Hydroxylgruppen oder

ii) das Derivat, worin eine oder mehrere der Hydroxylgruppen durch eine YR-Gruppe ersetzt sind, worin Y = O oder S ist, R = eine $C_1$-$C_6$-Alkyl-, $C_1$-$C_4$-Alkoxy-$C_2$-$C_5$-Alkyl-, $C_1$-$C_4$-Alkoxycarbonyl-$C_2$-$C_5$-alkyl-, Amino-$C_2$-$C_5$-alkyl-, Mercapto-$C_2$-$C_5$-alkyl-, Monohydroxy-$C_2$-$C_5$-alkyl-, Halogen-$C_2$-$C_5$-alkyl- oder $(R')_m$-Phenyl$(CH_2)_n$-Gruppe ist, worin R' eine $C_1$-$C_4$-Alkyl-, $C_1$-$C_4$-Alkoxy- oder Hydroxygruppe ist, m 0 bis 2 ist und n 0 bis 3 ist und wenn Y O ist, R auch eine 2,3-Dihydroxyprop-1-yl-Gruppe sein kann oder

iii) das $C_1$-$C_7$-Alkyl-, Aryl-$C_1$-$C_7$-alkyl- oder Halogenaryl-$C_1$-$C_7$-alkylesterderivat der Carboxylgruppe oder

iv) das Urethanderivat der A-Ring-Hydroxylgruppe, wobei das OH durch $OR^2$ ersetzt ist, worin $R^2$ eine $C_1$-$C_7$-Alkyl-, Aryl-, $C_1$-$C_7$-Alkylaryl-, Aryl-$C_1$-$C_7$-alkyl-, Halogenaryl-, Nitroaryl-,Halogenaryl-$C_1$-$C_7$-alkyl-, $C_1$-$C_7$-Alkoxyaryl-,Aryloxyaryl-, Aryl-$C_3$-$C_7$-cycloalkyl-, $C_1$-$C_7$-Acylaryl- oder $C_3$-$C_7$-Cycloalkylgruppe ist

oder ein Salz davon, wobei die Arylgruppe in einer der obigen Definitionen eine Phenyl-, Furyl- oder Pyridylgruppe sein kann.

2. Antibiotikum A80438 der Formel (1) gemäß Anspruch 1.

3. Natriumsalz des Antibiotikums A80438 der Formel (1) gemäß Anspruch 1.

4. Verbindung der Formel 1 nach einem der Ansprüche 1 bis 3 zur Verwendung zur Prävention oder Behandlung mikrobieller Infektionen.

5. Verbindung der Formel 1 nach einem der Ansprüche 1 bis 3 zur Verwendung als Wachstumsförderer.

6. Verfahren zur Herstellung einer Verbindung der Formel 1 nach einem der Ansprüche 1 bis 3, umfassend, daß man Streptomyces bobili NRRL 15971 oder Streptomyces pactum NRRL 15970 oder eine A80438 produzierende Variante oder Mutante davon in einem Kulturmedium, das assimilierbare Quellen für Kohlenstoff, Stickstoff und anorganische Salze enthält, unter aeroben Submersfermentations-Bedingungen züchtet, bis das Antibiotikum A80438 produziert wird und gegebenenfalls anschließend A80438 aus dem Kulturmedium abtrennt.

7. Streptomyces bobili NRRL 15971.

8. Streptomyces pactum NRRL 15970.

9. Geflügelfutter-Zusammensetzung, umfassend als erste Komponente eine Verbindung der Formel 1 nach einem der Ansprüche 1 bis 3 und eine zweite Komponente, die ausgewählt ist aus der Gruppe, bestehend aus

a) Nicarbazin,

b) 4,4'-Dinitrocarbanilid,

c) einem Naphthalinamin der Formel

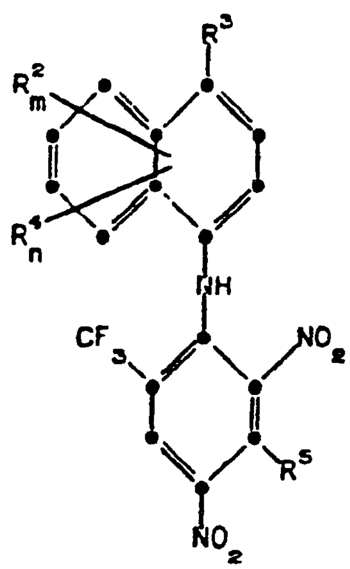

worin

$R^2$ eine $C_1$-$C_4$–Alkylgruppe ist;
$R^3$ Halogen, eine $C_1$-$C_4$–Fluoralkyl–, $C_1$-$C_4$–Fluor-alkoxy– oder $C_1$-$C_4$–Fluoralkylthiogruppe ist;
$R^4$ Halogen ist;
$R^5$ Wasserstoff oder Halogen ist;
m 0, 1 oder 2 ist und
n 0 oder 1 ist;
mit dem Vorbehalt, daß dann, wenn ein $R^4$-Substituent existiert, dieser an einer anderen als der 2-Position ist;

d) einem Benzolamin, ausgewählt aus 2,4-Dinitro-N-[4-(trifluormethoxy)-phenyl]-6-(trifluormethyl)-benzolamin, 2,4-Dinitro-N-[4-(1,1,2,2-tetrafluorethoxy)-phenyl]-6-(trifluormethyl)-benzolamin oder 2,4-Dinitro-N-[4-(pentafluorethoxy)-phenyl]-6-(trifluormethyl)-benzolamin;
e) Metichlorpindol oder
f) einem pharmazeutisch annehmbaren Salz einer der Verbindungen (a) bis (e); wobei die Komponenten in dem Futter in Mengen vorhanden sind, die in Kombination synergistisch wirken auf mindestens einen Coccidiose verursachenden Stamm von Eimeria.

**Ansprüche für folgenden Vertragsstaat : AT**

1. Verfahren zur Herstellung eines Antibiotikums A80438 der Formel 1 :

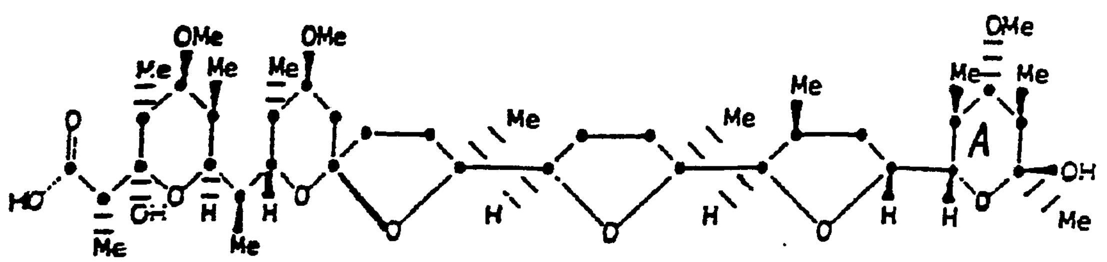

(1)

oder

i) des $C_1$-$C_7$-Acylesterderivats einer der Hydroxylgruppen oder
ii) des Derivats, worin eine oder mehrere der Hydroxylgruppen durch eine YR-Gruppe ersetzt sind, worin
Y = O oder S ist,
R = eine $C_1$-$C_6$–Alkyl–, $C_1$-$C_4$–Alkoxy–$C_2$-$C_5$–Alkyl–, $C_1$-$C_4$–Alkoxycarbonyl–$C_2$-$C_5$–alkyl–, Amino–$C_2$-$C_5$–alkyl–, Mercapto–$C_2$-$C_5$–alkyl–, Monohydroxy-$C_2$-$C_5$–alkyl–, Halogen–$C_2$-$C_5$–alkyl– oder $(R')_m$–Phenyl$(CH_2)_n$–Gruppe ist, worin R' eine $C_1$-$C_4$–Alkyl–, $C_1$-$C_4$–Alkoxy– oder Hydroxygruppe ist, m 0 bis 2 ist und n 0 bis 3 ist und wenn Y O ist, R auch eine 2,3-Dihydroxyprop-1-yl-Gruppe sein kann oder
iii) des $C_1$-$C_7$–Alkyl–, Aryl–$C_1$-$C_7$–alkyl– oder Halogenaryl–$C_1$-$C_7$–alkylesterderivats der Carboxylgruppe oder
iv) des Urethanderivats der A-Ring-Hydroxylgruppe, wobei das OH durch $OR^2$ ersetzt ist, worin $R^2$ eine $C_1$-$C_7$–Alkyl–, Aryl–, $C_1$-$C_7$–Alkylaryl–, Aryl–$C_1$-$C_7$–alkyl–, Halogenaryl–, Nitroaryl–, Halogenaryl–$C_1$-$C_7$–alkyl–, $C_1$-$C_7$–Alkoxyaryl–, Aryloxyaryl–, Aryl–$C_3$-$C_7$–cycloalkyl–, $C_1$-$C_7$–Acylaryl– oder $C_3$-$C_7$–Cycloalkylgruppe ist oder eines Salzes davon, wobei die Arylgruppe in einer der obigen Definitionen eine Phenyl–, Furyl– oder Pyridylgruppe sein kann, das umfaßt, daß man Streptomyces bobili NRRL 15971 oder Streptomyces pactum NRRL 15970 oder eine A80438-produzierende Variante oder Mutante davon in einem Kulturmedium, das assymilierbare Quellen für Kohlenstoff, Stickstoff und anorganische Salze enthält unter aeroben Submers-Fermentations-Bedingungen züchtet, bis das Antibiotikum A80438 produziert wird und gegebenenfalls anschließend A80438 aus dem Kulturmedium abtrennt.
2. Verfahren nach Anspruch 1 zur Herstellung von A80438 als Natriumsalz.

## Revendications

**Revendications pour les Etats contractants : BE, CH, DE, FR, GB, IT, LI, NL, SE**

1. Antibiotique A80438 de formule 1

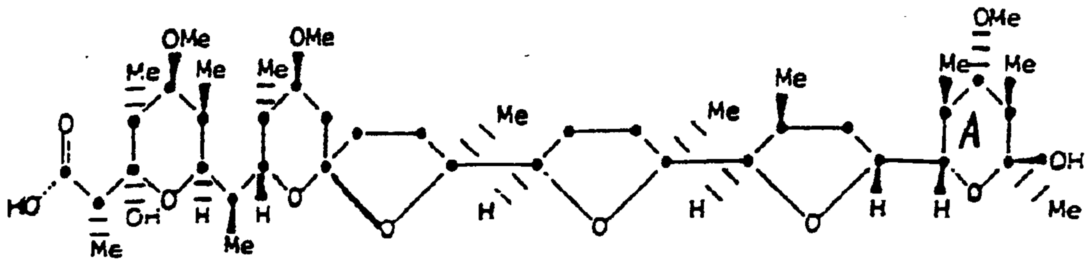

(1)

ou

i) le dérivé ester acylé en $C_1$-$C_7$ d'un des groupes hydroxyle ou

ii) le dérivé dans lequel un ou plusieurs des groupes hydroxyle a été remplacé par un groupe YR dans lequel :

Y représente O ou S ;

R représente un groupe alkyle en $C_1$-$C_6$, un groupe alcoxy(en $C_1$-$C_4$)alkyle en $C_2$-$C_5$, un groupe alcoxy(en $C_1$-$C_4$)carbonylalkyle en $C_2$-$C_5$, un groupe aminoalkyle en $C_2$-$C_5$, un groupe mercaptoalkyle en $C_2$-$C_5$, un groupe monohydroxy-alkyle en $C_2$-$C_5$, un groupe halogénalkyle en $C_2$-$C_5$ ou un groupe $(R')_m$–Phényl$(CH_2)_n$–, dans lequel R′ représente un groupe alkyle en $C_1$-$C_4$, un groupe alcoxy en $C_1$-$C_4$ ou un groupe hydroxyle ; m = 0-2 ; n = 0-3,

et lorsque Y = O, R peut également représenter un groupe 2,3-dihydroxyprop-1-yle ou

iii) le dérivé ester alkylique en $C_1$-$C_7$, arylalkylique en $C_1$-$C_7$ ou halogénarylalkylique en $C_1$-$C_7$ du groupe carboxyle ou

iv) le dérivé uréthanne du groupe hydroxyle à noyau A, dans lequel on remplace OH par $OR^2$ où $R^2$ =un groupe alkyle en $C_1$-$C_7$ un groupe aryle, un groupe alkyl(en $C_1$-$C_7$)aryle, un groupe arylalkyle en $C_1$-$C_7$, un groupe halogénaryle, un groupe nitro-aryle, un groupe halogénarylalkyle en $C_1$-$C_7$, un groupe alcoxy(en $C_1$-$C_7$)aryle, un groupe aryloxyaryle, un groupe arylcycloalkyle en $C_3$-$C_7$, un groupe acyl(en $C_1$-$C_7$)aryle ou un groupe cycloalkyle en $C_3$-$C_7$ ou encore

un de leurs sels, le groupe aryle pouvant représenter dans n'importe laquelle des définitions ci-dessus, un groupe phényle, un groupe furyle ou un groupe pyridyle.

2. Antibiotique A80438 de formule 1 selon la revendication 1.

3. Sel de sodium de l'antibiotique A80438 de formule 1 selon la revendication 1.

4. Composé de formule 1 selon l'une quelconque des revendications 1 à 3, destiné à être utilisé pour la prévention ou le traitement d'infections microbiennes.

5. Composé de formule 1 selon l'une quelconque des revendications 1 à 3, destiné à être utilisé comme stimulateur de croissance.

6. Procédé pour la préparation d'un composé de formule 1 selon l'une quelconque des revendications 1 à 3, consistant à cultiver **Streptomyces bobili** NRRL 15971 ou **Streptomyces pactum** NRRL 15970 ou encore un de leurs variants ou mutants producteurs de A80438, dans un milieu de culture contenant des sources assimilables de carbone, de l'azote et des sels inorganiques, dans des conditions aérobies immergées jusqu'à ce que l'on obtienne l'antibiotique A80438, cette mise en culture étant éventuellement suivie de la séparation de A80438 du milieu de culture.

7. **Streptomyces bobili** NRRL 15971.

8. **Streptomyces pactum** NRRL 15970.

9. Composition d'aliments pour volaille comprenant un premier constituant, à savoir un composé de formule 1 selon l'une quelconque des revendications 1 à 3, et un second constituant choisi parmi le groupe comprenant :

a) la nicarbazine ;

b) le 4,4′-dinitrocarbanilide ;

c) une naphtalène-amine de formule

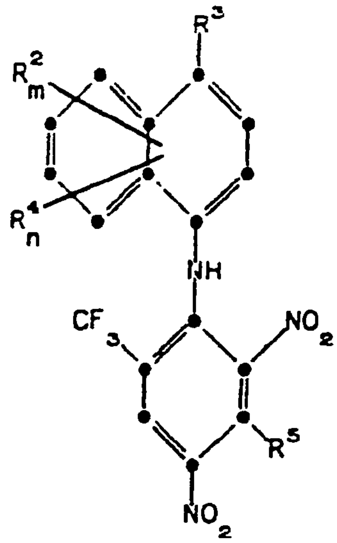

dans laquelle :

$R^2$ représente un groupe alkyle en $C_1$-$C_4$ ;

$R^3$ représente un atome d'halogène, un groupe fluoroalkyle en $C_1$-$C_4$, un groupe fluoroalcoxy en $C_1$-$C_4$ ou un groupe fluoroalkyl (en $C_1$-$C_4$) thio ;

$R^4$ représente un atome d'halogène ;

$R^5$ représente un atome d'hydrogène ou un atome d'halogène ;

m représente 0, 1 ou 2 ; et

n représente 0 ou 1 ; avec cette réserve que lorsqu'un substituant $R^4$ existe, il se trouve à une position différente de la position 2 ;

d) une benzène-amine choisie parmi la 2,4-dinitro-N-[4-(trifluorométhoxy)phényl]-6-(trifluoro-méthyl)benzène-amine ; la 2,4-dinitro-N-[4-(1,1,2,2-tétrafluoréthoxy)phényl]-6-(trifluorométhyl)benzène-amine ou la 2,4-dinitro-N-[4-(pentafluoréthoxy)-phényl]-6-(trifluorométhyl)benzène-amine ;

e) le métichloropindol ; ou

f) un sel pharmaceutiquement acceptable d'un composé (a)-(e) ;

les composants étant présents dans les aliments dans des quantités qui, en combinaison, ont un effet synergique contre au moins une souche d'**Eimeria** responsable de la coccidiose.

**Revendications pour l'Etat contractant suivant : AT**

1. Procédé pour la préparation de l'antibiotique A80438 de formule 1

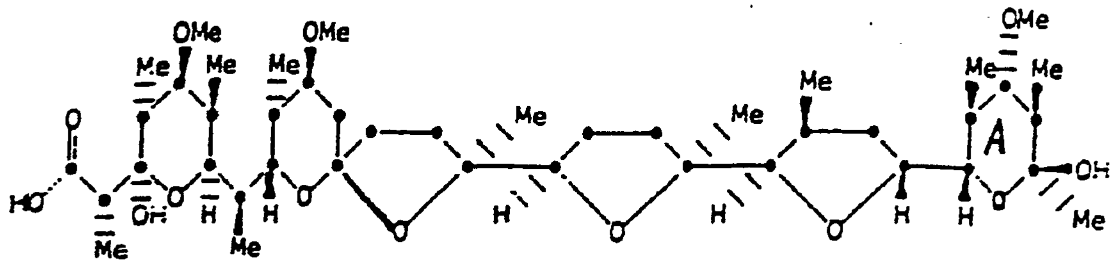

(1)

ou

i) du dérivé ester acylé en $C_1$-$C_7$ d'un des groupes hydroxyle ou

ii) du dérivé dans lequel un ou plusieurs des groupes hydroxyle a été remplacé par un groupe YR dans lequel :

Y représente O ou S ;

R représente un groupe alkyle en $C_1$-$C_6$, un groupe alcoxy(en $C_1$-$C_4$)alkyle en $C_2$-$C_5$, un groupe

alcoxy(en $C_1$-$C_4$)carbonylalkyle en $C_2$-$C_5$, un groupe aminoalkyle en $C_2$-$C_5$, un groupe mercaptoalkyle en $C_2$-$C_5$, un groupe monohydroxyalkyle en $C_2$-$C_5$, un groupe halogénalkyle en $C_2$-$C_5$ ou un groupe $(R')_m$-phényl$(CH_2)_n$-, dans lequel R' représente un groupe alkyle en $C_1$-$C_4$, un groupe alcoxy en $C_1$-$C_4$ ou un groupe hydroxyle ; m = 0-2 ; n = 0-3,

et lorsque Y = O, R peut également représenter un groupe 2,3-dihydroxyprop-l-yle ou

iii) du dérivé ester alkylique en $C_1$-$C_7$, arylalkylique en $C_1$-$C_7$ ou halogénarylalkylique en $C_1$-$C_7$ du groupe carboxyle ou

iv) du dérivé uréthanne du groupe hydroxyle à noyau A, dans lequel on remplace OH par $OR^2$ où $R^2$ =un groupe alkyle en $C_1$-$C_7$ un groupe aryle, un groupe alkyl(en $C_1$-$C_7$)aryle, un groupe arylalkyle en $C_1$-$C_7$, un groupe halogénaryle, un groupe nitro-aryle, un groupe halogénarylalkyle en $C_1$-$C_7$, un groupe alcoxy(en $C_1$-$C_7$)aryle, un groupe aryloxyaryle, un groupe arylcycloalkyle en $C_3$-$C_7$, un groupe acyl(en $C_1$-$C_7$)aryle ou un groupe cycloalkyle en $C_3$-$C_7$ ou encore

un de leurs sels, le groupe aryle pouvant représenter dans n'importe laquelle des définitions ci-dessus, un groupe phényle, un groupe furyle ou un groupe pyridyle, consistant à cultiver **Streptomyces bobili** NRRL 15971 ou **Streptomyces pactum** NRRL 15970 ou encore un de leurs variants ou mutants producteurs de A80438, dans un milieu de culture contenant des sources assimilables de carbone, de l'azote et des sels inorganiques, dans des conditions aérobies immergées jusqu'à ce que l'on obtienne l'antibiotique A80438, cette mise en culture étant éventuellement suivie de la séparation de A80438 du milieu de culture.

2. procédé selon la revendication 1 pour la préparation de A80438 sous la forme de son sel de sodium.

FIG.I

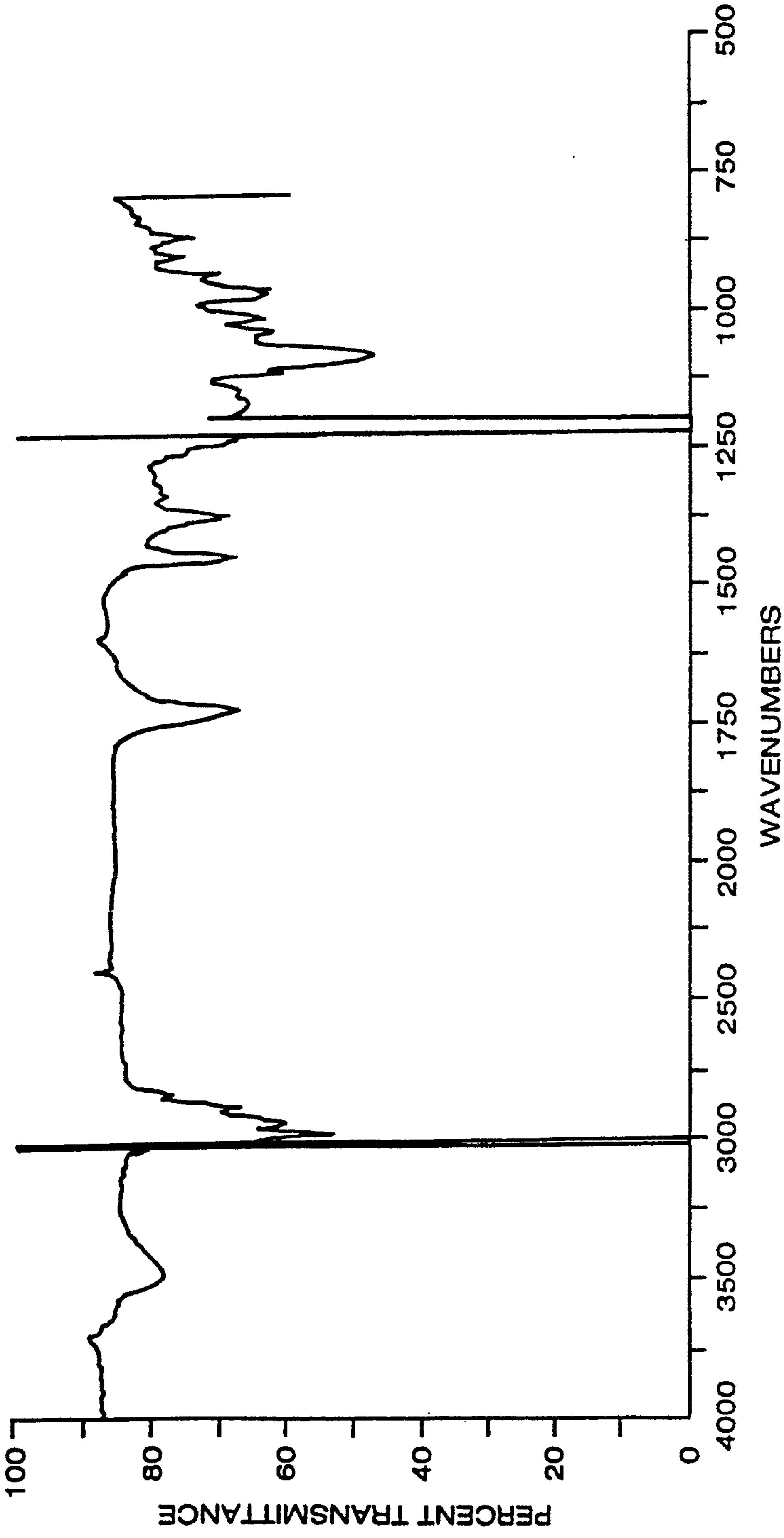
PERCENT TRANSMITTANCE
100
80
60
40
20
0
WAVENUMBERS
4000
3500
3000
2500
2000
1750
1500
1250
1000
750
500

FIG.2

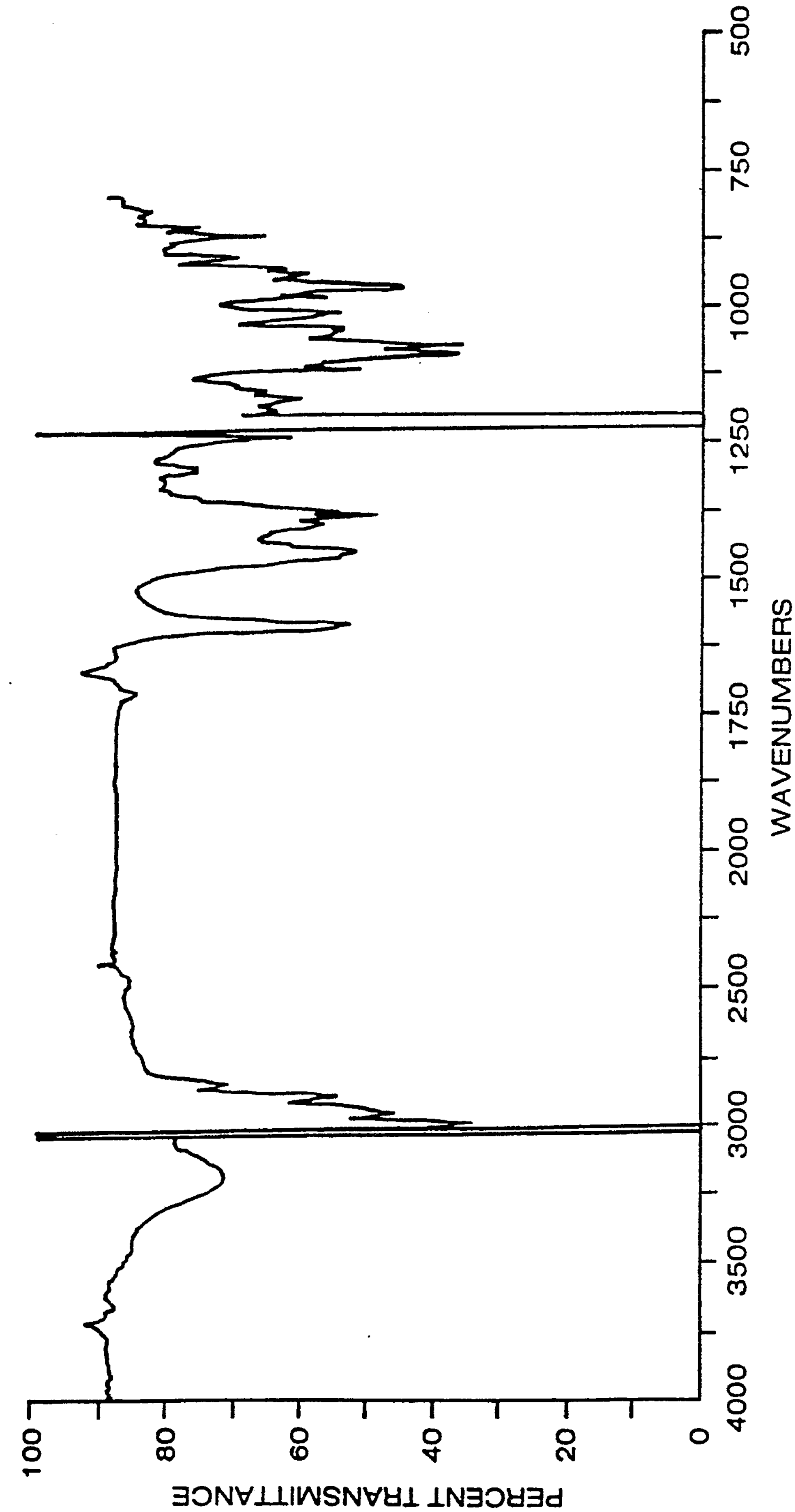
PERCENT TRANSMITTANCE
100
80
60
40
20
0
WAVENUMBERS
4000
3500
3000
2500
2000
1750
1500
1250
1000
750
500